# EUROPEAN PATENT SPECIFICATION

(11) **EP 2 211 802 B1**
(45) Date of publication and mention of the grant of the patent: **27.06.2012**
(21) Application number: 08843434.5
(22) Date of filing: 31.10.2008
(51) Int. Cl.: A61F 9/01

(54) **APPARATUS FOR IMPROVED POST-OPERATIVE OCULAR OPTICAL PEFORMANCE**
GERÄT FÜR VERBESSERTE POSTOPERATIVE OKULARE SEHLEISTUNG
APPAREILS D'AMELIORATION DES PERFORMANCES OPTIQUES APRES UNE OPERATION OPHTALMOLOGIQUE

(30) Priority: 02.11.2007 US 985192 P
(43) Date of publication of application: 04.08.2010
(73) Proprietor: Alcon LenSx, Inc., Aliso Viejo, CA 92656 (US)
(72) Inventor: KURTZ, Ronald M., Irvine California 92603 (US)
(74) Representative: Hanna, Peter William Derek
(86) International application number: PCT/US2008/082156
(87) International publication number: WO 2009/059251

(56) References cited:
- EP-A1- 1 252 872
- WO-A1-03/022168
- KR-A- 20020 093 935
- US-A- 5 968 095
- US-A1- 2004 199 149
- US-A1- 2006 235 428
- US-A1- 2007 173 759
- US-B2- 6 913 603

## Description

### Background

This application relates to laser eye surgery.

A number of different types of eye surgery can provide correction of refractive errors to improve near vision, distance vision, or both. While the surgical target of such surgeries may be specifically the cornea, the lens, or another particular eye tissue, such interventions typically have additional, inadvertent effects on the optical imaging in the eye. EP 1 252 872 A1 discloses an apparatus for tracking the movement of an eye during a diagnosis of the eye or during a corrective procedure such as corneal laser surgery

### Summary

Systems are described for increasing the control over the primary surgical intervention, while simultaneously decreasing the unintended side effects of this same surgery.

An exemplary method for improving an optical function of an eye includes preparing the eye for surgery by determining an optical characteristic of the eye. Laser marking pulses are applied to generate a laser mark in a region of the eye in relation to the determined optical characteristic. Also, a surgical procedure is performed in a surgical region selected in relation to the generated laser mark.

Implementations can optionally include one or more of the following features. The optical characteristic of the eye can be selected from one of an optical axis, a visual axis, a line of sight, a pupillary axis, and a compromise axis of the eye. Preparing the eye can include aligning the eye along the selected axis of the eye. The optical characteristic can include an orientation of the eye. Preparing the eye can include making a preparatory mark on an external region of the eye to reference the determined optical characteristic of the eye using at least one of an ink-based marker, a mechanical indentation of a cornea, an attachable marker and a laser pulse. Applying the laser marking pulses can include applying laser pulses to a target in a cornea or a lens of the eye in relation to the determined optical characteristic. Applying the laser pulses can include at least one of applying laser pulses to a point-like or circular target to indicate a selected axis applying laser pulses to a region removed from a center of the eye to mark an orientation of the eye. Applying the laser marking pulses can include applying the laser pulses when a patient is in a first patient position. Performing the surgical procedure can include performing the surgical procedure when the patient is in a second patient position. The first patient position can include an upright position to cause an eye of the patient to assume an essentially neutral or normal position. The second patient position can include a supine position to cause the eye to deviate from the neutral or normal position. Applying the laser pulses can include applying laser pulses to cause one of an incision and a perforation in at least one of a cornea, a lens, a lens capsule, and a limbus. Applying the laser pulses can include applying the laser pulses to two separate targets of the eye, the two targets being aligned in relation to the determined optical characteristic. Applying the laser pulses can include applying the laser pulses to the two separate targets comprising a corneal target and a lens target, wherein the corneal target and the lens target are aligned in relation to one of a selected axis of the eye; and a selected orientation of the eye. Performing the surgical procedure can include creating an incision on a lens capsule using a surgical laser; removing a portion of a natural lens from the capsule; inserting an intraocular lens into the lens capsule; and aligning the intraocular lens in the lens capsule according to laser marks generated to mark at least one of an axis of the eye, a center of the eye, and an orientation of the eye. Performing a surgical procedure can include placing a corneal inlay into the eye; and aligning the corneal inlay in the eye according to laser marks generated to mark at least one of an axis of the eye, a center of the eye, and an orientation of the eye. Performing the surgical procedure can include aligning an intraocular lens or a corneal inlay by aligning a complimentary mark of the intraocular lens or the corneal inlay with a laser mark.

In another aspect, an exemplary method for improving an optical function of an eye includes aligning the eye along a selected axis of the eye. Also, the method includes performing at least one of using a mark formed on an image of at least one part of the eye relative to the selected axis of the eye as a reference, and selecting an image of at least one part of the eye relative to the selected axis of the eye as a reference. A beam of laser pulses are controlled to direct the laser pulses to a target on the eye relative to the selected axis of the eye to coordinate the optical alignment of the post-operative eye.

In yet another aspect, a laser system for eye surgery includes a pulsed laser to produce a beam of laser pulses. The system includes a mechanism to align an eye along a selected axis of the eye. Also, the system includes an imaging module to monitor at least one of positioning of a reference mark made on or in the eye, and a selected reference image of the at least one part of the eye relative to the selected axis of the eye. The system includes a control module to communicate with the imaging module to direct the laser pulses to at least one target in the eye relative to the selected axis of the eye.

Implementations can optionally include one or more of the following features. The system can include a mechanism to align the eye is configured to fix a position of the eye relative to the selected axis. The imaging module is designed to monitor a position of the reference mark and a position of an anatomic reference. The reference mark is placed when the eye is in a non-applanated state. The anatomic reference is determined when the eye is in an applanated state.

In yet another aspect, an exemplary method to align multiple optically impactful procedures on an eye includes selecting an axis on which to align at least one of the optically impactful procedures. A physical mark can be made on an external region of the eye to represent a position of the selected axis. The physical mark is identified and displayed on an image of the eye. Also, a position of the identified physical mark on the image of the eye is used to direct laser pulses to at least one target in the eye relative to the mark or the selected axis of the eye.

Implementations can optionally include one or more of the following features. Laser-marks generated by laser pulses can be used to guide a subsequent alignment. Corneal incisions and ablations can be aligned. The laser-marks can be used to guide alignment or placement of intraocular or intra-corneal implants. A mark can be made on or in an eye by applying laser pulses. The mark can be identified. Also, at least one of choosing and confirming an optical element to be implanted in the eye can be performed. Information about the identified mark can be used to assist a predicting of a position of an intraocular lens to be implanted in the eye.

In yet another implementation, a laser system for eye surgery can include a pulsed laser to produce a beam of laser pulses. The system includes an imaging module to monitor a position of a reference mark made by laser pulses on at least one target in the eye.

Implementations can optionally include one or more of the following features. The system can include a computer system to calculate a recommended intraocular lens power based at least in part on the position of the reference mark. At least one of the pulsed laser, the imaging module and the computer system can be designed to use a mark on an intraocular lens or corneal inlay in conjunction with the reference mark to improve one of a positioning, a choice of an optical power or size of the intraocular lens or corneal inlay.

### Brief Description of the Drawings

FIG. 1 shows main optical elements of an eye.

FIGs. 2A-B shows example surgical methods. These methods are not a part of the invention and are described for illustrative purposes.

FIG. 3 shows an example process for preparing an eye.

FIG. 4 shows an example process for determining a visual axis and preparatory marking and laser-marking.

FIG. 5 shows insertion of an intraocular lens as part of a surgical process.

FIGs. 6A-B shows example frontal views of an eye for surgical preparation.

FIG. 7 shows an example of an imaging-guided laser surgical system including an imaging module to provide imaging of a target for laser control.

FIGs. 8-16 show examples of imaging-guided laser surgical systems with varying degrees of integration of a laser surgical system and an imaging system.

FIG. 17 shows an example process for performing laser surgery by using an imaging-guided laser surgical system.

FIG. 18 shows an example image of an eye from an optical coherence tomography (OCT) imaging module.

FIGs. 19A, 19B, 19C and 19D show two examples of calibration samples for calibrating an imaging-guided laser surgical system.

FIG. 20 shows an example of attaching a calibration sample material to an interface in an imaging-guided laser surgical system for calibrating a system.

FIG. 21 shows an example of reference marks created by a surgical laser beam on a glass surface.

FIG. 22 shows an example of the calibration process and the post-calibration surgical operation for an imaging-guided laser surgical system.

FIGs. 23A and 23B show two operation modes of an exemplary imaging-guided laser surgical system that captures images of laser-induced photodisruption byproduct and the target issue to guide laser alignment.

FIGs. 24 and 25 show examples of laser alignment operations in imaging-guided laser surgical systems.

FIG. 26 shows an exemplary laser surgical system based on the laser alignment using the image of the photodisruption byproduct.

### Detailed Description

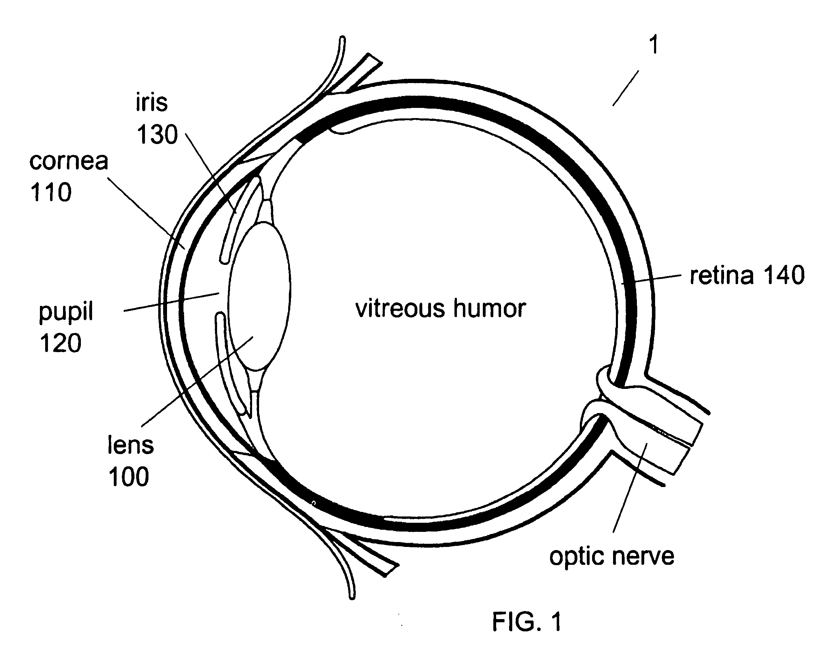

FIG. 1 shows an eye 1. The incident light propagates through the optical path which includes the cornea 110, the anterior chamber, the pupil 120, defined by the iris 130, the lens 100, the posterior chamber, and the vitreous humor. These optical elements guide the light on the retina 140.

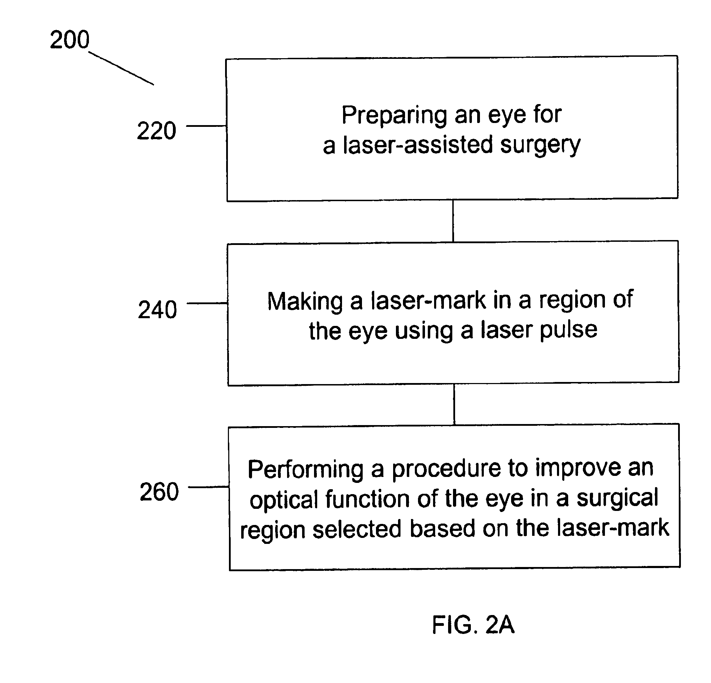

FIG. 2A shows an example process 200 for performing an eye surgical procedure. In step 220 an eye is prepared for a laser assisted surgery. In step 240 a laser-mark is made in a region of the eye using a laser pulse, and in step 260 a surgical procedure is performed to improve an optical property of the eye in a surgical region selected in relation to the laser-mark.

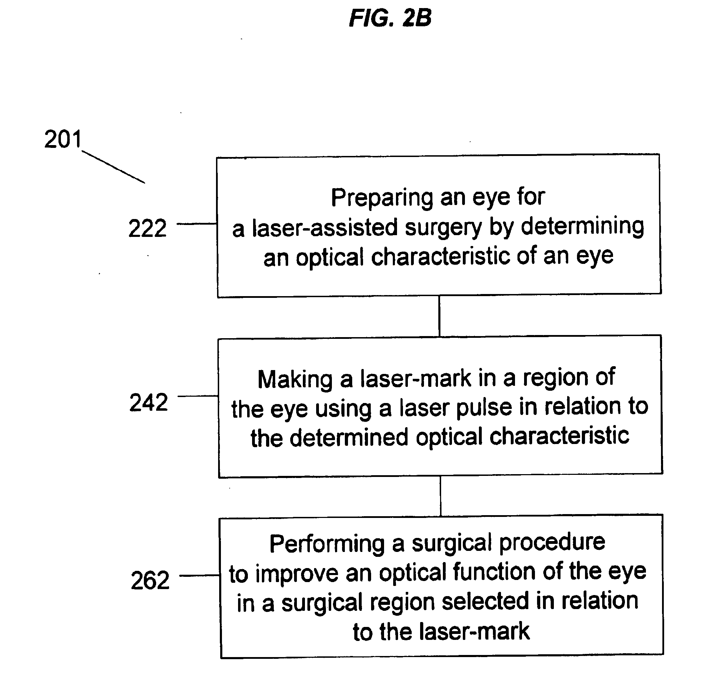

FIG. 2B shows another example process 201 for performing an eye surgical procedure. In step 222 an eye is prepared for a laser assisted surgery by determining an optical characteristic of the eye. In step 242 a laser-mark is made in a region of the eye using a laser pulse in relation to the determined optical characteristic. In step 262 a surgical procedure is performed to improve an optical property of the eye in a surgical region selected in relation to the laser-mark.

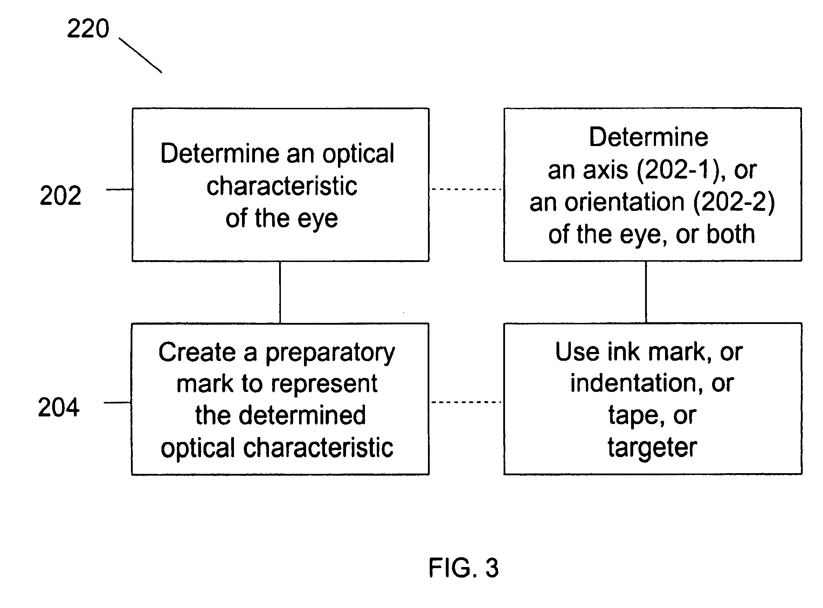

FIG. 3 shows that preparatory step 220 may involve a step 202 of determining an optical characteristic of the eye. The optical characteristic can be any specific feature, point, or identifier of the eye.

An example of such a characteristic is an axis of the eye. Other examples include an uppermost point of the pupil when the patient is in an upright position, as outlined below.

The axes of the eye can be described in several different manners and any one of these descriptions of the eye axes can be utilized in step 202. The axes of the eye can be categorized e.g. according to the Grand Y.L. Physiological Optics (Springer-Verlag, New York, 1980) as follows:
Optical axis: Line passing through the optical center of the cornea and the lens;
Visual axis: Line passing from the point of fixation to the image on the center of the retina called fovea;
Line of Sight: Line passing from the object point through the center of the entrance of the pupil; and
Pupillary axis: Line passing perpendicularly through the center of the cornea and the center of the entrance of the pupil.

These axes can be described as theoretical axes, geometrical axes, functional axes, anatomical axes, or any combination of the above.

These axes of the eye are typically close to each other, or aligned with each other, but do not necessarily coincide. For example, the optical center of the cornea and the center of the lens, the two major refractive elements of the eye, are generally not naturally aligned relative to the geometrical center of the eye. Often the center of the lens is slightly nasal to that of the cornea. In addition, the center of the pupil is generally not aligned with the axes connecting any two of the centers of the cornea, the lens, and the fovea. The centers of these optical elements can be misaligned by as much as 500 microns or more. In step 202-1 any one of these axes can be identified. Alternatively, the surgeon can identify a compromise axis, which lies in between any two or more selected axes.

Various surgical equipments (described below in detail) may be attached to the eye on which the surgery will be performed. Then, the patient is asked to concentrate on a target with the other eye. The knowledge of the point of fixation is then used to identify the visual axis of the eye on which the surgery is to be performed, even though that eye does not necessarily see the point of fixation. Alternatively, fixation of the surgical eye can be accomplished and the orientation of the eye directly identified during such alignment.

Preparatory step 220 may also include step 204, in which a preparatory mark is made to represent the determined characteristic of the eye. An example is to use an ink marker to make a preparatory mark on the surface of the eye where the eye axis, selected in step 202-1, intersects the surface of the eye.

Other examples include using a corneal epithelial marker, or an indentation on the surface of the eye (without using ink), or attaching any type of physical markers, such as a small piece of adhesive tape. Any combination of these markers can be used as well.

Some existing devices include a targeter. The surgeon can point the targeter to an appropriately chosen point of the eye, such as its center, or align a circle of the targeter with a circular feature of the eye, such as the pupil. Then the applicator, formed as a unit with the targeter, applies ink to the center, as defined by the targeted center or the circular feature.

Preparatory step 220 may include step 202-2, where the optical characteristic of the eye represents an orientation of the eye in a first patient position.

For example, although most eye surgeries are performed with the patient in the supine position with the eye looking up, they often involve a preparatory step, when the patient is in a first patient position, e.g. sitting up. The above preparatory steps 202 - 204 can be performed in this sitting-up position to record the position of features of the eye in this position. Then the surgical steps, such as step 260, are performed the patient being in a second patient position, such as in a supine position, looking up.

Quite often the eye of the patient rotates when the patient is moved from the upright first position to the laying down second position. In some cases the eye can rotate as much as 10-60 degrees. This is the so-called "cyclo-rotation", or "cyclo-torsion". If the surgical steps do not correct for this rotation relative to the preparatory steps, the surgical procedure may be performed in a location different from the optimal, possible leading to a clinically relevant reduction of the efficacy of the surgery.

Preparatory step 202-2 can include making a preparatory mark with any of the above described marking methods to indicate an orientation of the eye ball. Using an analogy with the face of a clock, a preparatory mark can be made e.g. at the three o'clock location in the first patient position. Then, when the patient is prepared for surgery by assuming the second patient position, e.g. lying down, the surgeon can examine the preparatory mark and infer the extent of the rotation of the eye ball from the deviation of the preparatory mark from the 3 o'clock location. The surgeon can then make the necessary adjustment with the knowledge of this rotation. These adjustments can include adjusting for refractive errors, both in astigmatism and in asymmetric higher order aberrations that are not symmetric around the pupil.

In a simple example, when refractive surgery is to be performed on the cornea, the patient can be asked to fixate on an appropriate target. The position of the image of the center of the pupil can be marked with ink or an indentation on the cornea to estimate the line of sight. If performed in the upright position, marks can also be made in the peripheral cornea, or limbus, to register the torsional state, or orientation, of the eye, to serve as reference when the patient is supine in subsequent surgical steps. Alternatively, the position of the image of the center of the pupil can be marked with ink or an indentation on the cornea overlying the center of the cornea as indicated by the corneal light reflex (Purkingee image), thus approximating the pupillary axis.

While the above methods can be used in a number of surgeries, including limbal relaxing incisions, astigmatic keratotomy and radial keratotomy, surface markings can potentially introduce errors due to the optical effects of the cornea and the location of the lens/pupil diaphragm several millimeters away. For example, during procedures that take more than a few seconds, such as corneal laser refractive procedures like PRK and LASIK, the pupil can rotate significantly relative to corneal marks and to the perceived image of the pupil, potentially introducing significant tilt errors to any surgical intervention. To overcome this, pupil trackers that monitor the movement of the pupil can be used. The most accurate of these can utilize sophisticated image processing to monitor the position of specific iris features. An example is the iris registration method, which is described in U.S. Patent 7,044,602.

The optimal alignment of the optical elements in the course of intraocular surgeries presents another set of challenges. In such procedures, the actual target of the procedure, the lens of the eye for instance, cannot be marked physically without entering the eye. If the surgeon relies on surface marks, those can be misleading, as the light propagation between the lens and the surface is actively impacted by the optical elements, such as the cornea. Further, even if some level of compensation for these impacts is introduced based e.g. on a software model of the eye, it is quite challenging to incorporate the fact that the eye itself can alter its shape, position and/or fixation. Finally, the pupil can dilate during such intraocular procedures, making the desired compensation based on registration landmarks even harder.

Until recently, the above described limitations were largely theoretical, since the intraocular surgeries did not demand as high a precision in centering and alignment. For example, until recently, cataract or lens removal surgery was used primarily to remove an aged natural lens and replace it with an artificial lens (intraocular lens, IOL) that had greater light transmission. Until recently these intraocular lenses were monofocal, requiring a precision which was attainable with the existing surface marking methods.

However, cataract surgery developed into one of the most commonly performed ophthalmic procedures worldwide and its goals have expanded. At present, cataract surgery is also used to improve the refractive functioning of the eye. In fact, lens removal and replacement is now commonly performed when little to no cataract is present, and refractive or optical correction is the primary goal.

Current lens surgery goals include reducing a person's dependence on glasses and other optical aids both for distant and near vision. Specific lens replacement procedures include the introduction of multifocal IOL's that provide two working distances for the eye. Examples include the Restore products, developed by Alcon and the Rezoom products, developed by AMO Inc. Another class of IOLs can move or change shape in the eye. Examples of such accommodating IOLs include Crystalens, developed by Eyeonics. IOLs with apertures that increase depth of field are also being developed, as evidenced by the ACI-7000, developed by Acufocus. In addition, IOLs that can correct higher order aberrations, such as the Light Adjustable Lens by Calhoun Vision, as well as those that correct astigmatism have also been developed. Finally, specialized low vision intraocular lenses have also been introduced to provide magnification for eyes with diseased retinas.

When compared with standard cataract surgery using monofocal IOLs, current lens surgery has become a complicated procedure, capable of addressing multiple vision correction goals. In contrast to standard monofocal IOLs, the optics of these newer devices is more sensitive to errors in centration and tilt. Existing methods to properly center these devices use cumbersome templates that must be physically inserted into the eye and have not gained favor.

The just-described aspects of modem cataract surgery indicate that qualitatively higher precision is required in the course of these procedures than in traditional eye surgery. In some cases a centering precision of less than 500 microns can be required when placing the IOLs. Errors exceeding this value may lead to a large aberration and qualitatively reduce the effectiveness e.g. of the multi-focal feature of the IOLs.

Another challenge is posed by surgeries which place an IOL into the lens by creating a cut, or incision, along a portion of the periphery of the cornea, and the lens. These incisions can cause unintended deformations of the cornea. In an example, using the above clock-face analogy for identifying the orientations, if the incision to introduce the IOL was made in the 12 o'clock region, the cornea may get flatter in the 12 - to - 6 o'clock direction ("meridian") and bulge in the 3 - to - 9 o'clock direction. This unintended side-effect can be used to correct for pre-existing astigmatic error by placing the incision in a particular meridian. Alternatively, pre-operative astigmatism can also be compensated by making additional incisions on the periphery at different meridian locations and for different arc lengths. These cuts are sometimes referred to as "limbal relaxing incisions", or "astigmatic keratotomy".

This was just one example of integrated eye surgical procedures, when more than one optical aspect of the eye is improved within a single surgical procedure. While such combined procedures offer the potential for enhanced outcomes, they also have the potential to introduce unwanted side effects due to unanticipated combined optical effects. For example, placement of a multifocal intraocular lens combined with performing an astigmatic keratotomy can provide a patient with good distance and near vision by concurrently addressing presbyopia, spherical and astigmatic refractive error. However, if the optical center of the multifocal lens is not aligned well with respect to the corneal correction, or if either correction is not well aligned with respect to one of the primary axes of the eye, an optical aberration may occur.

Similarly, a LASIK procedure, aimed at a distance refractive correction, can include the insertion of a corneal inlay to improve near vision. Such a LASIK procedure may enhance the optical performance of the eye. However, if the center of the LASIK procedure is not well aligned with respect the corneal inlay, or either manipulation is not well aligned with respect to one of the axes of the eye, an optical aberration may occur, causing visual symptoms.

While two examples have been described so far, a number of other possible procedure combinations can be envisioned to interfere constructively or destructively, including combination procedures that are entirely contained within the cornea or lens. Such combinations can also include corneal inlays, such as the Intacs products, developed by Addition Technology, or presbyopic inlays, such as inlays developed by Revision Optics, and the ACI-7000, developed by Acufocus, as well as artificial pupils placed in front of, or behind, a natural or artificial lens. While each individual procedure can be aligned when performed separately, the multiple required alignments are time consuming and may not result in optimized performance when combined.

The above discussion has focused on more advanced developments in refractive and cataract surgery. However, higher precision alignment can be advantageous even in the most common technique for cataract and lens removal, in ultrasound based phaco-emulsification. In phaco-emulsification, a relatively small corneal incision is made. Through this incision, a gel like substance is injected into the eye to maintain intraocular pressure and the form of the anterior chamber. In a procedure called capsulotomy, an opening is formed in the anterior capsule using various mechanical techniques or equivalents. Next, an ultrasound probe is introduced into the eye through this opening. This ultrasound probe is used to fragment the native lens, which is subsequently removed via aspiration through the corneal incision. An intraocular lens is then inserted through the same corneal incision.

Since this technique uses a relatively small incision in the cornea, the surgically induced astigmatism is limited. However, a fundamental limitation of phaco-emulsfication arises from its step-wise protocol: the multiple, independent surgical manipulations of the procedure. These manipulations, including the corneal incision, capsulotomy, lens fragmentation and removal, and the introduction of the intraocular lens lead to optimal results when each of them are properly aligned with respect to the eye's axes and anatomy.

In any of the above procedures an increased precision of the alignment of the successive surgical steps can lead to an increase of the likelihood that the procedures result in the desired improvement of the optical function of the eye, while reducing the negative effects.

The improved alignment methods for ocular refractive procedures include tracking the cyclo-torsion and other ocular movements that can occur between the preparatory marking and the surgical procedure or between individual surgical steps, tracking and correcting for parallax and other optical effects, and coordinating multiple separate interventions conveniently and efficiently to optimize total post-operative optical performance.

Examples of the methods 200 and 201 provide such techniques by performing a laser marking step 240 in conjunction with the preparatory marking step 204. Performing two marking steps can increase an accuracy and precision of the subsequent surgical procedure 260 by more closely linking the steps of alignment, marking and surgery, no matter where on the eye a refractive procedure is performed.

Laser pulses, when applied with the appropriate intensity, repetition rate and energy per pulse, can create small gas bubbles in a tissue of the eye. These bubbles can be used for forming a laser-mark in the tissue. In contrast to the preparatory marking in step 204, typically applied on the surface of the eye, these laser-generated bubbles can be generated inside the tissue, directly in the target region. Therefore, they can mark the target region with a precision unhindered by optical distortion, or a subsequent shape-change of the eyeball when the surgical procedure begins. Such direct, or in-depth, laser marking therefore can be quantitatively superior to the preparatory surface marking. As described above, several of the modern eye surgical methods benefit considerably from this improved precision of the laser-marking step 240.

In one implementation of the eye surgical methods 200 and 201, a surgical system can include a mechanism to identify at least one axis of the eye, such as the eye's visual axis in step 202-1. The system can also include a mechanism to correlate the position of the identified axis relative to an anatomic landmark, such as the limbus, cornea, lens capsule and lens, in an orientation marking step 202-2. Finally, the system may include a mechanism to create a preparatory mark on an anatomic structure in the eye in step 204, to guide the application of the subsequent laser marking step 240 and the surgical intervention step 260 to improve an optical function of the eye.

In operation of the above system, an axis of the eye can be identified in the step 202-1. For example, the visual axis may be identified by a number of methods, including the use of a fixation target. Such axis identification may be performed during a normal functioning of the eye in the body, such as with the eye looking forward at a distant or near fixation target with the head in the upright position. In alternative examples the patient may be instructed to assume alternative orientations, such as gaze slightly downward as in reading, or alternative positions, such as a supine position, with the eye looking up. Alternatively, another axis of the eye may be identified, or a combination of axes and anatomic information can be used to determine a compromise axis with which to align planned surgical procedures.

Following the determination of the selected axis of the eye, or a compromise axis, a preparatory marking can be made to record the relationship between the identified axis and the anatomy of the eye in step 204. The recordation can be made by making preparatory markings on the surface of the eye. Or, the position of the determined axis can be recorded within a three dimensional image of the eye produced via ultrasound, optical or other means and captured during appropriate positioning of the eye (during fixation for example) in an imaging device.

In the subsequent step 240, laser bubbles can be generated in an internal target region of the eye, such as the limbus, cornea, lens capsule and/or lens, to create laser-marks. The generation of the laser-marks can be assisted by, among others, the preparatory markings. The generated laser-marks can indicate the position of the target region for the subsequent surgical procedure 260 with high precision. More than one target region may be laser-marked. E.g. a primary target can be laser-marked for the insertion of an IOL and a secondary target can be laser-marked for a limbal relaxing incision, to counteract astigmatism, inadvertently caused by the primary incision.

The laser-marking can be of different extent. The laser can be used to generate the marking bubbles with high density. A sufficiently high density of bubbles can perforate a tissue, enabling the surgeon to subsequently separate the perforated tissue via tearing, cutting, aspiration or some other intervention in the surgical step 260. The properly placed perforation can ensure that the alignment and other aspects of the surgical intervention, including the size, shape and location of the removed or separated tissue, are appropriate for the surgical goal. Surgical step 260 can use lasers or any other type of surgical tools, including mechanical and other devices.

The laser-marks can serve as aids for subsequent surgical manipulations, such as for guiding the placement of an intraocular lens (IOL) or a corneal inlay. In such cases, the laser-marks can include scores, partial or full depth cuts or holes or other physical effects that can guide the surgical placement of the IOL or the corneal inlay. The laser marking can also serve additional functions, such as to aid a fixation, stabilization or orientation of the IOL or the corneal inlay. In addition, the laser marks can guide post-operative procedures performed to enhance optical performance, such as by reducing residual post-operative astigmatism via extension of a limbal relaxing incision along the laser mark.

Finally, the laser pulses themselves can serve a dual purpose: being both markers for marking step 240, as well as inducing the desired surgical effect itself in the step 260, such as the fragmentation, cutting or vaporizing of specific structures such as the lens capsule or any portion of the lens. Using lasers for both in-depth marking as well as for the surgery itself can ensure a high precision of the alignment, shape, size, location and other characteristics of the surgical process and target region relative to the axes of the eye.

The application of lasers can include a trephination of the lens capsule which can be asymmetric or symmetric to allow both access to lens tissue and optimal orientation of the IOL relative to the visual, or other axis. In this example, centration and orientation of the capsulotomy and thus the IOL can be accomplished in an integrated procedure, without the need for additional manual manipulation, in contrast to some techniques, such as the one described by Maskett in U.S. Patent Application Publication No. 20040106929.

In another example, laser pulses can be applied in the cornea/limbus to create partial or full depth corneal perforations in step 240. By separating the tissues in the appropriate regions, properly aligned, sized and positioned corneal incisions can be created for use in various interventions in step 260, including creating self sealing entry cuts for the removal and replacement of the crystalline lens, creating transverse corneal incisions or limbal relaxing incisions to treat pre-existing or surgically induced astigmatism, or creating corneal flaps or beds for subsequent LASIK or corneal inlay procedures.

Additional examples are also possible, including combination laser procedures in the lens, lens capsule, cornea and limbus. In one implementation, marks or registrations can be first created on the cornea and then used for subsequent manipulations inside the eye such as to orient the position of a capsulotomy procedure, or the placement of an IOL.

In another implementation, laser-marks or registrations can be first generated on the lens capsule, with a subsequent corneal surgical procedure 260 performed, its orientation guided by these intraocular marks. Such corneal procedures can include procedures that require no additional corneal manipulation, such as intrastromal procedures to correct ammetropia or presbyopia.

The above and other implementations of the exemplary methods and apparatus allow for an enhanced, in some cases optimal precision for laser eye surgery procedures in an eye relative to functional and anatomic features of the eye, thereby optimizing its optical functionality. This improvement may be accomplished in an integrated method, using one device so that the overall procedure may be simplified. Alternate examples may practice the method in separate steps.

Optimal axis determination and imaging in step 202-1 may require a stabilization or fixation of the eye in a particular position, for example during visual fixation or when the eye is being imaged. In addition, laser pulse placement in the eye may require fixation or applanation of the eye. The relative positions of the determined axes of the eye and the anatomical parts of the eye may thus be shifted from their neutral or natural positions when the eye is not fixated and/or applanated. In such cases, the relationship between the positions of the axes of the eye, and positions of the anatomical parts of the eye in the neutral and post-fixation and/or post-applanation state may be compared and registered so that the surgical intervention in step 260 can be performed in such a manner that the surgically altered features have the correct orientation when the eye is returned to the neutral state.

An image of the eye may be created in the first patient position, with the preparatory markings also registered. Then, in the second patient position the markings are once again recorded, and the torsional or other changes in the shape and orientation of the eye are determined. These data are then used by software to calculate the optimal location for the surgical intervention in the second position such that the surgically altered optical elements will acquire their desired shape when the patient returns into the first patient position.

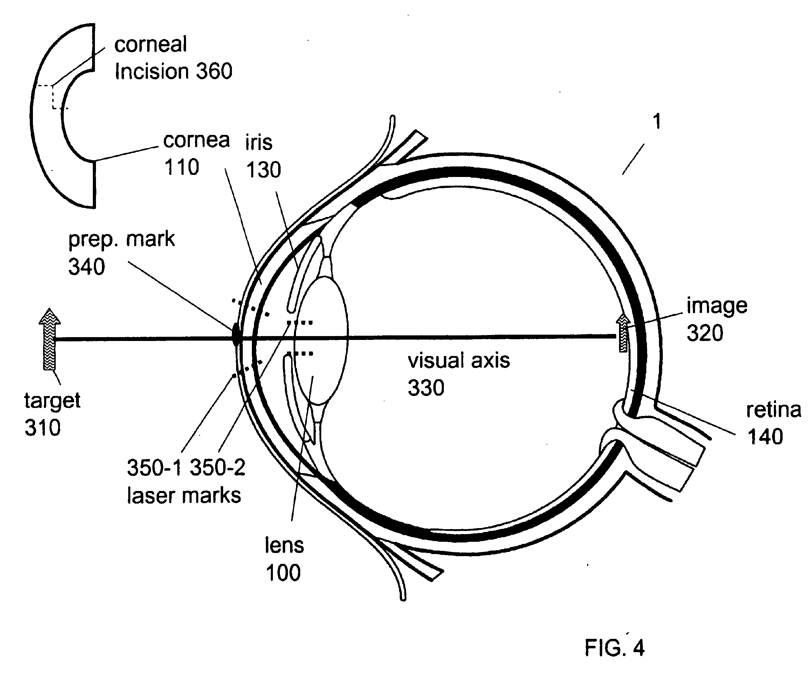

FIG. 4 shows that in an exemplary embodiment of step 202-1 the visual axis 330 can be determined by aligning the target of fixation 310 with the image 320 on the center of the retina.

In step 204, a preparatory marking 340 (not to scale) can be made on the outside of the cornea where the visual axis intercepts the cornea.

In step 240, laser pulses can be applied to the lens and cornea to create laser mark perforations (corneal marks 350-1 and capsular marks 350-2) that are aligned with respect to the visual axis as well as to each other. The laser marks/perforations 350 can be a group or line of bubbles. The laser pulses can be directed by utilizing the preparatory marking 340 of step 204.

In step 260, the surgeon can use the corneal perforation 350-1 as a guide for the entry incision into the eye, thereby allowing for precise, laser guided control of the shape of the incision, its location, size and orientation, in order to optimize its structure and function. The magnified inset shows that, for example, a corneal entry incision 360 can be designed to be multilevel to provide a self-sealing incision. Alternatively, the entry incision can be positioned to affect corneal astigmatism.

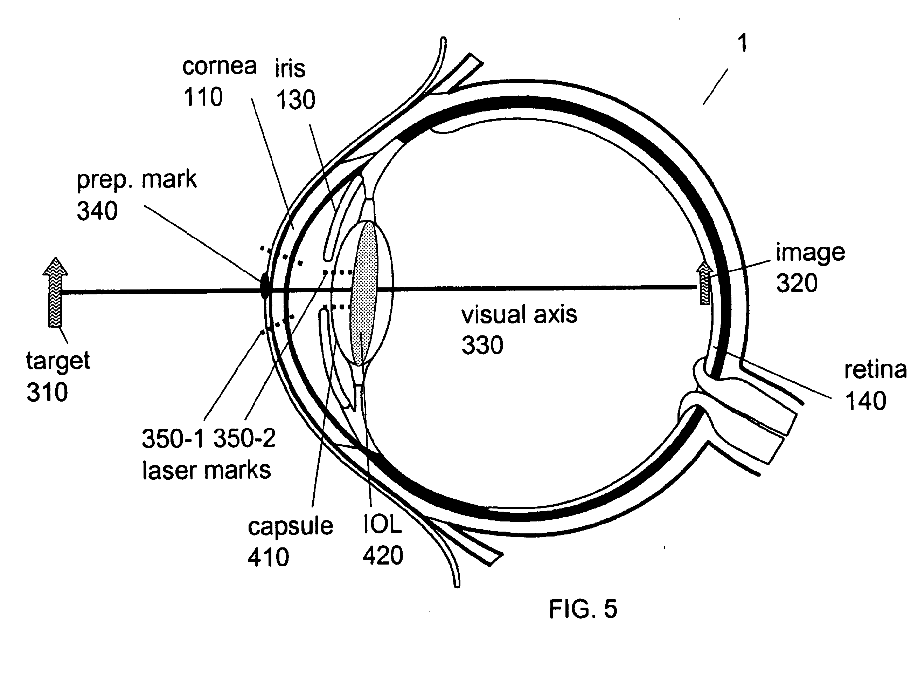

FIG. 5 shows that the capsular marks 350-2 can assist the surgeon to create an appropriately sized, positioned and oriented capsulotomy. The capsular marks 350-2 can be subsequently used to guide a placement of an IOL 420 into the capsular bag 410 with high precision and alignment with respect to the visual axis 330, as well as to the corneal mark/perforation 350-1.

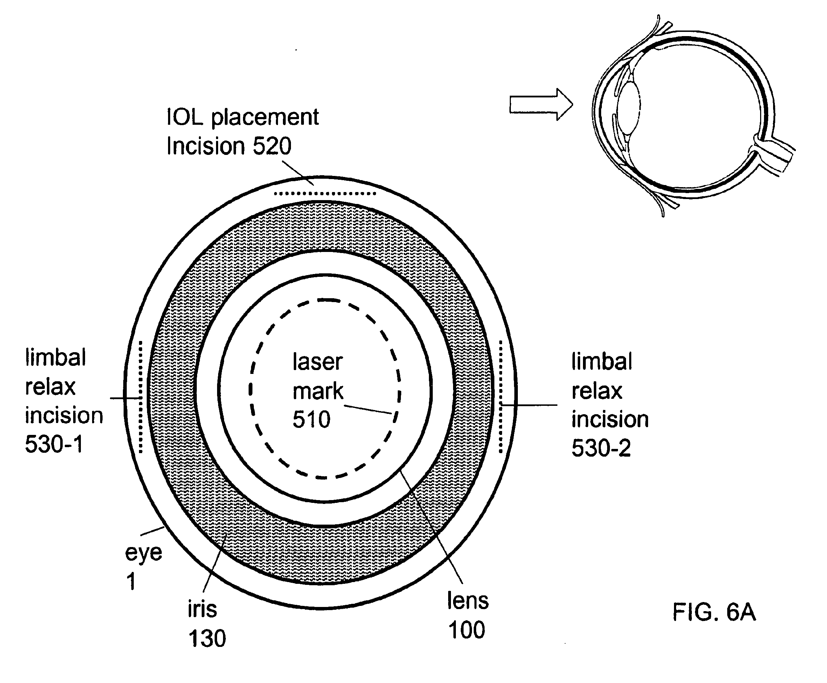

FIGs. 6A-B show the markings and incisions in an analogous embodiment from a frontal view, as indicated by the arrow of the inset. FIG. 6A shows that laser marks 510, analogous to laser marks 350 of FIG. 5, can be placed e.g. concentrically on the cornea 110, lens 100 or capsule 410. In surgical step 260 e.g. an IOL placement incision 520 can be made, for example, at the 12 o'clock position of the cornea 110 and capsule 410. The positioning of the IOL placement incision 520 can be guided by previously placed laser marks 350 (not shown). As mentioned earlier, the laser can be operated first to generate the laser marks, then to generate the surgical laser pulses.
The same laser can be used first with a low energy density pulses to generate laser-marks, then with high energy density pulses to generate surgical incisions.

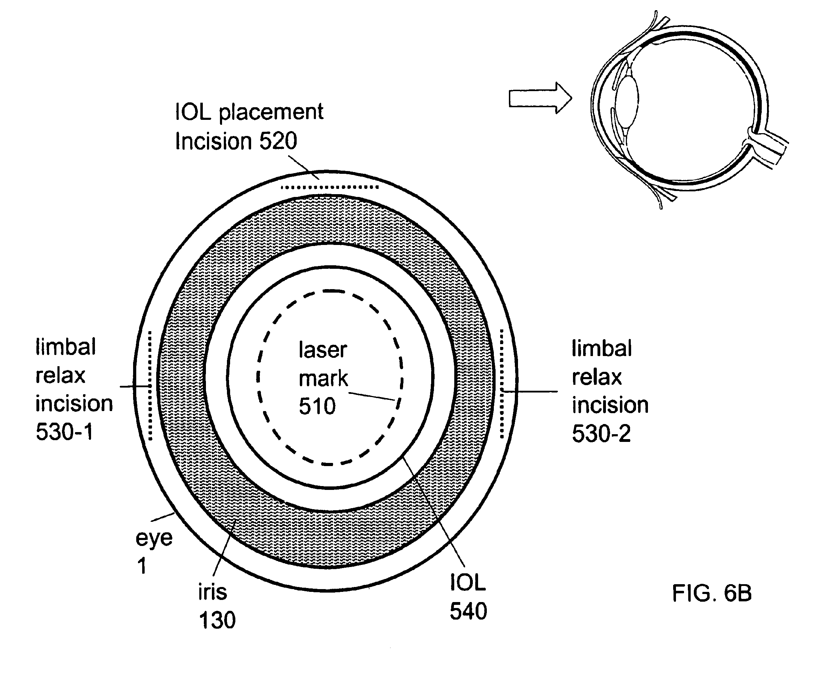

FIG. 6B shows that during the surgical step 260 an intraocular lens 540 can be inserted into the lens capsule 410 through the IOL placement incision 520. Using the laser marks 510, placed during the laser marking step 240, the IOL 540 can be aligned with the visual axis of the eye and centered with high precision.

Returning to FIG. 6A, the surgeon may want to additionally compensate the generation of unwanted or pre-existing astigmatism by making limbal relaxing incisions. Additional laser marks (not shown) can be placed e.g. at the 3 o'clock and 9 o'clock positions. These laser marks can be then used during the surgical step to place the limbal relaxing incisions 530-1 and 530-2 with proper alignment and high precision. The limbal relaxing incisions can be straight, curved or arced, depending on additional considerations.

FIG. 6B shows the insertion of the IOL 540 again aligned and centered using the laser marks 510.

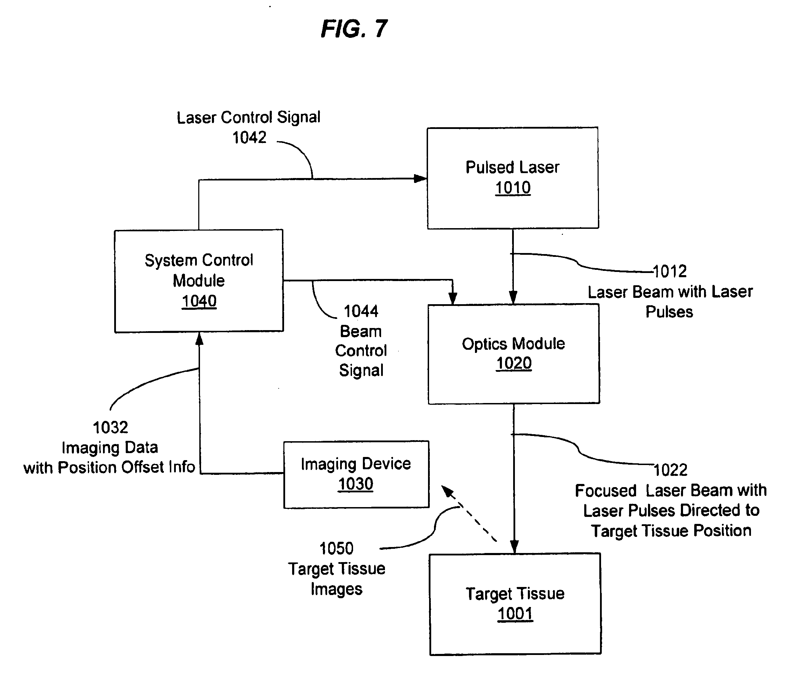

A laser eye surgical system in relation to the above surgical methods 200 and 201 will be described in detail in the context of FIGs. 7-26. In operation of the surgical system, an eye can be aligned relative to an optics module with a fixation module. An image of the eye can be captured with an imaging module. In step 220, an axis or compromise axis can be determined by the surgical system control module. In step 240 a laser-mark can be made on at least one part of the eye or an image of the eye relative to the determined axis. The mark can be a real physical mark on the eye or it can be a virtual mark on the image or eye.

In the case of the former, the laser-mark may be formed by one or more laser pulses directed onto the eye by the surgery system control module. Alternatively, or in conjunction with such a marking, the eye may be physically fixed in its orientation relative to the determined axis. Such fixation can be accomplished by several means including use of a limbal suction ring or fixation teeth. In another alternative, the selected axis of the eye can be determined and marked completely with the use of the laser system. A physical mark on the cornea or some other external portion of the eye can be identified by one or more imaging modalities of the surgical system and be represented on the image used to guide laser treatment.

In step 260, the surgeon can be guided by the position of the laser-mark to ensure proper identification of the eye axis. For the targeting of the surgical procedure, the surgeon can accept, modify or over-ride the laser-mark, relying on additional information. Once the surgeon determines the final target positions, laser pulses can be directed to the target in the eye, e.g. to cut or create an incision or a perforation. Since the eye has been marked or oriented with respect to the selected axis, such incisions or perforations are also aligned relative to the selected axis.

In the case where the incision or perforation itself has an optical effect (either alone or in conjunction with additional surgical manipulations, such as manual tearing along the laser perforation), then the optical effects caused by the incision are also aligned with the selected axis. If manipulations are made at more than one optical surface or element, for example in the lens and cornea, then each of the manipulations can be aligned to the selected axis and thus with each other. In addition to direct effects mediated by the laser pulses, additional optical elements, such as cornea or lens implants can be aligned to the selected axis, or compromise axis, subsequent to the laser-markings, incisions or perforations.

In some examples the surgical procedures 260 are performed when the eye is in a state different from its normal or neutral orientation or physical state. For example, the usual position of the patient is upright for most tasks, while supine positioning is required or preferred for most surgical procedures. In addition, the cornea is generally free from applanation, while some laser procedures are preferentially performed with the cornea applanated. When the patient and eye are placed in these other orientations or states, marks can be placed which may move relative to the desired axis or rotational orientation. Thus to optimize post-operative optical performance there is also a need to correlate the marks made in one state with the state of the eye during the actual surgical procedure.

A surgeon marks or indicate one or more positions on the cornea when the patient is upright and the cornea is not applanated. When the patient is then put in the supine position and the cornea is applanated, the position of these marks can be identified by the surgeon or by an imaging system of the surgical system. In either case, the position of the marks can be displayed on a treatment image to help direct the placement of the surgical laser pulses that either affect optical performance of the eye themselves, or which themselves guide surgical steps that affect optical performance of the eye.

Surgical laser pulses that are placed, or the incisions or marks made by these surgical pulses can be imaged intraoperatively to help guide selection of optical implants to be placed in the eye. For example, a mark or cut of the lens capsule can be imaged to identify the anterior chamber depth, a key input for proper intraocular lens selection.

A patient may sit in an upright position during the preparatory step 220. A surgeon may identify an axis in the eye during the step 202-1, and make a preparatory mark with ink in the step 204 where the identified axis intersects the cornea, as well as a preparatory mark at the 3 o'clock location on the limbal region, i.e. the periphery of the cornea, to mark the orientation of the eye.

Next, in a step 240, the patient is escorted to the surgery room, where she is instructed to lie down in a supine position. The surgeon may place a fixation ring on the eye to be treated, to suppress the movement of the patient's eye during the surgical procedure. The surgeon may also place a contact lens on the eye for the purpose of applanation. Making the surface of the eye more flat increases the surgeon's ability to target the surgical laser pulses to the optimal region with a high precision.

Next, an image of the eye, including the cornea and the lens can be taken by a computer controller system. The image may indicate e.g. that the preparatory mark at 3 o'clock rotated to about the 4 o'clock direction, i.e. by about 30 degrees, when the patient assumed the supine position.

Next, the software of the controller system can calculate where the eventual surgical intervention should be targeted to, so that when the patient resumes her normal, upright position, the treated region will rotate back where its location was intended. For example, the controller system may have selected an appropriate pattern of incisions from a database of stored patterns, or may have tailored a pattern for the patient, both based on the preparatory markings and the image just taken. Then the controller may identify where the surgical procedure needs to be targeted on the eye, while the patient is in the supine position so that when the eye rotates back to its normal orientation, the incisions will be where desired.

The surgeon may then apply laser pulses to create laser-marks on the cornea and the lens, to assist e.g. the insertion of an IOL.

The surgeon may follow the recommendation of the controller directly, or can modify it to a limited degree, or can overrule it to a considerable degree on the basis of e.g. individual judgment.

The laser-marks may be used only to align and orient the subsequent surgical steps 260 relative to the visual axis and the natural rotational state of the eye. The surgical steps 260 may include the application of more powerful laser pulses, possibly with higher repetition rate, or any non-laser based surgical procedure, including any variants of the phaco-emulsification approach.

The laser-marks can also play a more prominent role in the surgical step 260. The laser-marks may be used to perforate the cornea and lens tissue at the properly aligned and rotated target location. In these implementations, the subsequent surgical steps 260 may include the surgeon removing unwanted tissue along the perforation via the laser-marks.

Such a removal step may be followed by a variety of additional surgical steps. For example a limited removal step may not have been the ultimate goal of the surgical procedure: such a limited removal could have been part of the subsequent formation of a layered cut in the cornea or lens.

Next, technical details of a laser surgical system will be described with various features for performing the processes described in this application.

As an example, FIG. 7 shows a laser surgical system based on optical imaging and applanation. This system includes a pulsed laser 1010 to produce a surgical laser beam 1012 of laser pulses, and an optics module 1020 to receive the surgical laser beam 1012 and to focus and direct the focused surgical laser beam 1022 onto a target tissue 1001, such as an eye, to cause photodisruption in the target tissue 1001. An applanation plate can be provided to be in contact with the target tissue 1001 to produce an interface for transmitting laser pulses to the target tissue 1001 and light coming from the target tissue 1001 through the interface. Notably, an optical imaging device 1030 is provided to capture light 1050 carrying target tissue images 1050 or imaging information from the target tissue 1001 to create an image of the target tissue 1001. The imaging signal 1032 from the imaging device 1030 is sent to a system control module 1040. The system control module 1040 operates to process the captured images from the image device 1030 and to control the optics module 1020 to adjust the position and focus of the surgical laser beam 1022 at the target tissue 101 based on information from the captured images. The optics module 120 can include one or more lenses and may further include one or more reflectors. A control actuator can be included in the optics module 1020 to adjust the focusing and the beam direction in response to a beam control signal 1044 from the system control module 1040. The control module 1040 can also control the pulsed laser 1010 via a laser control signal 1042.

The optical imaging device 1030 may be implemented to produce an optical imaging beam that is separate from the surgical laser beam 1022 to probe the target tissue 1001 and the returned light of the optical imaging beam is captured by the optical imaging device 1030 to obtain the images of the target tissue 1001. One example of such an optical imaging device 1030 is an optical coherence tomography (OCT) imaging module which uses two imaging beams, one probe beam directed to the target tissue 1001 thought the applanation plate and another reference beam in a reference optical path, to optically interfere with each other to obtain images of the target tissue 1001. In other implementations, the optical imaging device 1030 can use scattered or reflected light from the target tissue 1001 to capture images without sending a designated optical imaging beam to the target tissue 1001. For example, the imaging device 1030 can be a sensing array of sensing elements such as CCD or CMS sensors. For example, the images of photodisruption byproduct produced by the surgical laser beam 1022 may be captured by the optical imaging device 1030 for controlling the focusing and positioning of the surgical laser beam 1022. When the optical imaging device 1030 is designed to guide surgical laser beam alignment using the image of the photodisruption byproduct, the optical imaging device 1030 captures images of the photodisruption byproduct such as the laser-induced bubbles or cavities. The imaging device 1030 may also be an ultrasound imaging device to capture images based on acoustic images.

The system control module 1040 processes image data from the imaging device 1030 that includes the position offset information for the photodisruption byproduct from the target tissue position in the target tissue 1001. Based on the information obtained from the image, the beam control signal 1044 is generated to control the optics module 1020 which adjusts the laser beam 1022. A digital processing unit can be included in the system control module 1040 to perform various data processing for the laser alignment.

The above techniques and systems can be used deliver high repetition rate laser pulses to subsurface targets with a precision required for contiguous pulse placement, as needed for cutting or volume disruption applications. This can be accomplished with or without the use of a reference source on the surface of the target and can take into account movement of the target following applanation or during placement of laser pulses.

The applanation plate in the present systems is provided to facilitate and control precise, high speed positioning requirement for delivery of laser pulses into the tissue. Such an applanation plate can be made of a transparent material such as a glass with a predefined contact surface to the tissue so that the contact surface of the applanation plate forms a well-defined optical interface with the tissue. This well-defined interface can facilitate transmission and focusing of laser light into the tissue to control or reduce optical aberrations or variations (such as due to specific eye optical properties or changes that occur with surface drying) that are most critical at the air-tissue interface, which in the eye is at the anterior surface of the cornea. A number of contact lenses have been designed for various applications and targets inside the eye and other tissues, including ones that are disposable or reusable. The contact glass or applanation plate on the surface of the target tissue is used as a reference plate relative to which laser pulses are focused through the adjustment of focusing elements within the laser delivery system relative. Inherent in such an approach are the additional benefits afforded by the contact glass or applanation plate described previously, including control of the optical qualities of the tissue surface. Accordingly, laser pulses can be accurately placed at a high speed at a desired location (interaction point) in the target tissue relative to the applanation reference plate with little optical distortion of the laser pulses.

The optical imaging device 1030 in FIG. 7 captures images of the target tissue 1001 via the applanation plate. The control module 1040 processes the captured images to extract position information from the captured images and uses the extracted position information as a position reference or guide to control the position and focus of the surgical laser beam 1022. This imaging-guided laser surgery can be implemented without relying on the applanation plate as a position reference because the position of the applanation plate tends to change due to various factors as discussed above. Hence, although the applanation plate provides a desired optical interface for the surgical laser beam to enter the target tissue and to capture images of the target tissue, it may be difficult to use the applanation plate as a position reference to align and control the position and focus of the surgical laser beam for accurate delivery of laser pulses. The imaging-guided control of the position and focus of the surgical laser beam based on the imaging device 1030 and the control module 1040 allows the images of the target tissue 1001, e.g., images of inner structures of an eye, to be used as position references, without using the applanation plate to provide a position reference.

In addition to the physical effects of applanation that disproportionably affect the localization of internal tissue structures, in some surgical processes, it may be desirable for a targeting system to anticipate or account for nonlinear characteristics of photodisruption which can occur when using short pulse duration lasers. Photodisruption can cause complications in beam alignment and beam targeting. For example, one of the nonlinear optical effects in the tissue material when interacting with laser pulses during the photodisruption is that the refractive index of the tissue material experienced by the laser pulses is no longer a constant but varies with the intensity of the light. Because the intensity of the light in the laser pulses varies spatially within the pulsed laser beam, along and across the propagation direction of the pulsed laser beam, the refractive index of the tissue material also varies spatially. One consequence of this nonlinear refractive index is self-focusing or self-defocusing in the tissue material that changes the actual focus of and shifts the position of the focus of the pulsed laser beam inside the tissue. Therefore, a precise alignment of the pulsed laser beam to each target tissue position in the target tissue may also need to account for the nonlinear optical effects of the tissue material on the laser beam. The energy of the laser pulses may be adjusted to deliver the same physical effect in different regions of the target due to different physical characteristics, such as hardness, or due to optical considerations such as absorption or scattering of laser pulse light traveling to a particular region. In such cases, the differences in non-linear focusing effects between pulses of different energy values can also affect the laser alignment and laser targeting of the surgical pulses. In this regard, the direct images obtained from the target issue by the imaging device 1030 can be used to monitor the actual position of the surgical laser beam 1022 which reflects the combined effects of nonlinear optical effects in the target tissue and provide position references for control of the beam position and beam focus.

The techniques, apparatus and systems described here can be used in combination of an applanation plate to provide control of the surface shape and hydration, to reduce optical distortion, and provide for precise localization of photodisruption to internal structures through the applanated surface. The imaging-guided control of the beam position and focus described here can be applied to surgical systems and procedures that use means other than applanation plates to fix the eye, including the use of a suction ring which can lead to distortion or movement of the surgical target.

The following sections first describe examples of techniques, apparatus and systems for automated imaging-guided laser surgery based on varying degrees of integration of imaging functions into the laser control part of the systems. An optical or other modality imaging module, such as an OCT imaging module, can be used to direct a probe light or other type of beam to capture images of a target tissue, e.g., structures inside an eye. A surgical laser beam of laser pulses such as femtosecond or picosecond laser pulses can be guided by position information in the captured images to control the focusing and positioning of the surgical laser beam during the surgery. Both the surgical laser beam and the probe light beam can be sequentially or simultaneously directed to the target tissue during the surgery so that the surgical laser beam can be controlled based on the captured images to ensure precision and accuracy of the surgery.

Such imaging-guided laser surgery can be used to provide accurate and precise focusing and positioning of the surgical laser beam during the surgery because the beam control is based on images of the target tissue following applanation or fixation of the target tissue, either just before or nearly simultaneously with delivery of the surgical pulses. Notably, certain parameters of the target tissue such as the eye measured before the surgery may change during the surgery due to various factor such as preparation of the target tissue (e.g., fixating the eye to an applanation lens) and the alternation of the target tissue by the surgical operations. Therefore, measured parameters of the target tissue prior to such factors and/or the surgery may no longer reflect the physical conditions of the target tissue during the surgery. The present imaging-guided laser surgery can mitigate technical issues in connection with such changes for focusing and positioning the surgical laser beam before and during the surgery.

The present imaging-guided laser surgery may be effectively used for accurate surgical operations inside a target tissue. For example, when performing laser surgery inside the eye, laser light is focused inside the eye to achieve optical breakdown of the targeted tissue and such optical interactions can change the internal structure of the eye. For example, the crystalline lens can change its position, shape, thickness and diameter during accommodation, not only between prior measurement and surgery but also during surgery. Attaching the eye to the surgical instrument by mechanical means can change the shape of the eye in a not well defined way and further, the change can vary during surgery due to various factors, e.g., patient movement. Attaching means include fixating the eye with a suction ring and applanating the eye with a flat or curved lens. These changes amount to as much as a few millimeters. Mechanically referencing and fixating the surface of the eye such as the anterior surface of the cornea or limbus does not work well when performing precision laser microsurgery inside the eye.

The post preparation or near simultaneous imaging in the present imaging-guided laser surgery can be used to establish three-dimensional positional references between the inside features of the eye and the surgical instrument in an environment where changes occur prior to and during surgery. The positional reference information provided by the imaging prior to applanation and/or fixation of the eye, or during the actual surgery reflects the effects of changes in the eye and thus provides an accurate guidance to focusing and positioning of the surgical laser beam. A system based on the present imaging-guided laser surgery can be configured to be simple in structure and cost efficient. For example, a portion of the optical components associated with guiding the surgical laser beam can be shared with optical components for guiding the probe light beam for imaging the target tissue to simplify the device structure and the optical alignment and calibration of the imaging and surgical light beams.

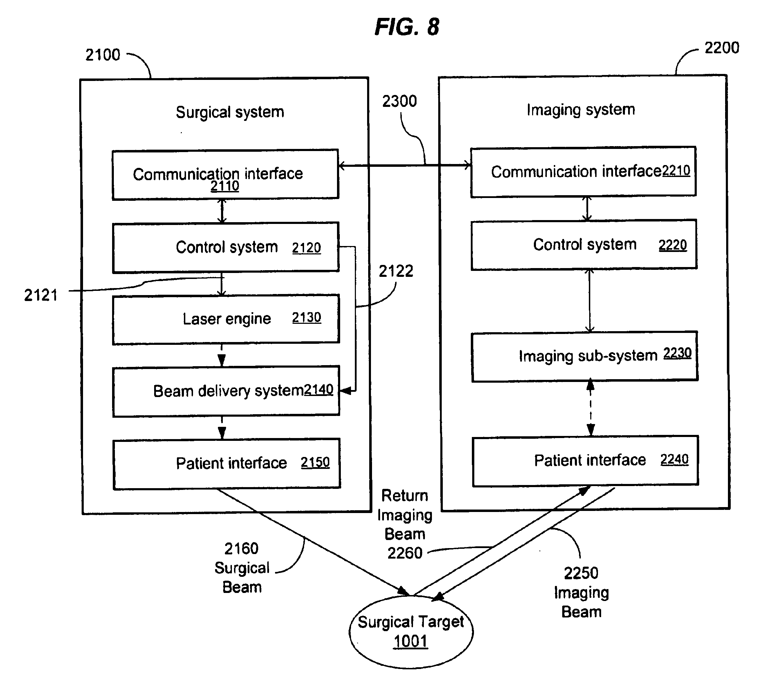

The imaging-guided laser surgical systems described below use the OCT imaging as an example of an imaging instrument and other non-OCT imaging devices may also be used to capture images for controlling the surgical lasers during the surgery. As shown in the examples below, integration of the imaging and surgical subsystems can be implemented to various degrees. In the simplest form without integrating hardware, the imaging and laser surgical subsystems are separated and can communicate to one another through interfaces. Such designs can provide flexibility in the designs of the two subsystems. Integration between the two subsystems, by some hardware components such as a patient interface, further expands the functionality by offering better registration of surgical area to the hardware components, more accurate calibration and may improve workflow. As the degree of integration between the two subsystems increases, such a system may be made increasingly cost-efficient and compact and system calibration will be further simplified and more stable over time. Examples for imaging-guided laser systems in FIGs. 8-16 are integrated at various degrees of integration.

One implementation of a present imaging-guided laser surgical system, for example, includes a surgical laser that produces a surgical laser beam of surgical laser pulses that cause surgical changes in a target tissue under surgery; a patient interface mount that engages a patient interface in contact with the target tissue to hold the target tissue in position; and a laser beam delivery module located between the surgical laser and the patient interface and configured to direct the surgical laser beam to the target tissue through the patient interface. This laser beam delivery module is operable to scan the surgical laser beam in the target tissue along a predetermined surgical pattern. This system also includes a laser control module that controls operation of the surgical laser and controls the laser beam delivery module to produce the predetermined surgical pattern and an OCT module positioned relative to the patient interface to have a known spatial relation with respect to the patient interface and the target issue fixed to the patient interface. The OCT module is configured to direct an optical probe beam to the target tissue and receive returned probe light of the optical probe beam from the target tissue to capture OCT images of the target tissue while the surgical laser beam is being directed to the target tissue to perform an surgical operation so that the optical probe beam and the surgical laser beam are simultaneously present in the target tissue. The OCT module is in communication with the laser control module to send information of the captured OCT images to the laser control module.

In addition, the laser control module in this particular system responds to the information of the captured OCT images to operate the laser beam delivery module in focusing and scanning of the surgical laser beam and adjusts the focusing and scanning of the surgical laser beam in the target tissue based on positioning information in the captured OCT images.

In some implementations, acquiring a complete image of a target tissue may not be necessary for registering the target to the surgical instrument and it may be sufficient to acquire a portion of the target tissue, e.g., a few points from the surgical region such as natural or artificial landmarks. For example, a rigid body has six degrees of freedom in 3D space and six independent points would be sufficient to define the rigid body. When the exact size of the surgical region is not known, additional points are needed to provide the positional reference. In this regard, several points can be used to determine the position and the curvature of the anterior and posterior surfaces, which are normally different, and the thickness and diameter of the crystalline lens of the human eye. Based on these data a body made up from two halves of ellipsoid bodies with given parameters can approximate and visualize a crystalline lens for practical purposes. In another implementation, information from the captured image may be combined with information from other sources, such as pre-operative measurements of lens thickness that are used as an input for the controller.

FIG. 8 shows one example of an imaging-guided laser surgical system with separated laser surgical system 2100 and imaging system 2200. The laser surgical system 2100 includes a laser engine 2130 with a surgical laser that produces a surgical laser beam 2160 of surgical laser pulses. A laser beam delivery module 2140 is provided to direct the surgical laser beam 2160 from the laser engine 2130 to the target tissue 1001 through a patient interface 2150 and is operable to scan the surgical laser beam 2160 in the target tissue 1001 along a predetermined surgical pattern. A laser control module 2120 is provided to control the operation of the surgical laser in the laser engine 2130 via a communication channel 2121 and controls the laser beam delivery module 2140 via a communication channel 2122 to produce the predetermined surgical pattern. A patient interface mount is provided to engage the patient interface 2150 in contact with the target tissue 1001 to hold the target tissue 1001 in position. The patient interface 2150 can be implemented to include a contact lens or applanation lens with a flat or curved surface to conformingly engage to the anterior surface of the eye and to hold the eye in position.

The imaging system 2200 in FIG. 8 can be an OCT module positioned relative to the patient interface 2150 of the surgical system 2100 to have a known spatial relation with respect to the patient interface 2150 and the target issue 1001 fixed to the patient interface 2150. This OCT module 2200 can be configured to have its own patient interface 2240 for interacting with the target tissue 1001. The imaging system 2200 includes an imaging control module 2220 and an imaging sub-system 2230. The sub-system 2230 includes a light source for generating imaging beam 2250 for imaging the target 1001 and an imaging beam delivery module to direct the optical probe beam or imaging beam 2250 to the target tissue 1001 and receive returned probe light 2260 of the optical imaging beam 2250 from the target tissue 1001 to capture OCT images of the target tissue 1001. Both the optical imaging beam 2250 and the surgical beam 2160 can be simultaneously directed to the target tissue 1001 to allow for sequential or simultaneous imaging and surgical operation.

As shown in FIG. 8, communication interfaces 2110 and 2210 are provided in both the laser surgical system 2100 and the imaging system 2200 to facilitate the communications between the laser control by the laser control module 2120 and imaging by the imaging system 2200 so that the OCT module 2200 can send information of the captured OCT images to the laser control module 2120. The laser control module 2120 in this system responds to the information of the captured OCT images to operate the laser beam delivery module 2140 in focusing and scanning of the surgical laser beam 2160 and dynamically adjusts the focusing and scanning of the surgical laser beam 2160 in the target tissue 1001 based on positioning information in the captured OCT images. The integration between the laser surgical system 2100 and the imaging system 2200 is mainly through communication between the communication interfaces 2110 and 2210 at the software level.

In this and other examples, various subsystems or devices may also be integrated. For example, certain diagnostic instruments such as wavefront aberrometers, corneal topography measuring devices may be provided in the system, or pre-operative information from these devices can be utilized to augment intra-operative imaging.

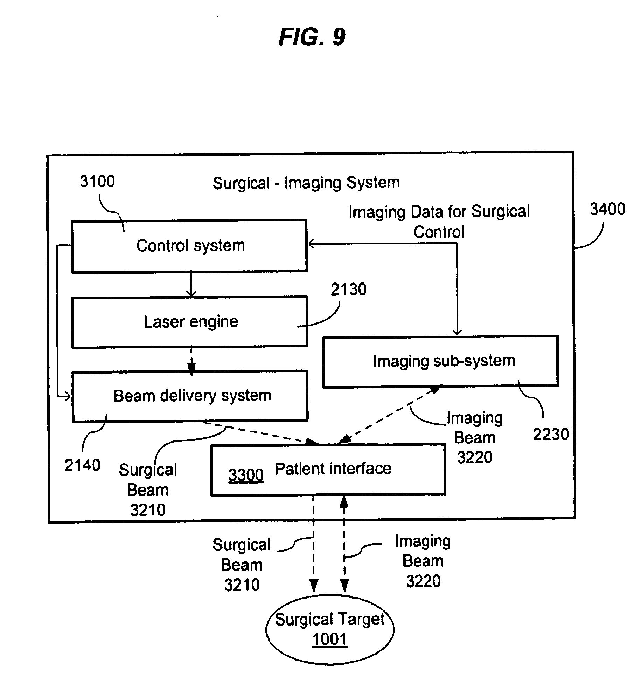

FIG. 9 shows an example of an imaging-guided laser surgical system with additional integration features. The imaging and surgical systems share a common patient interface 3300 which immobilizes target tissue 1001 (e.g., the eye) without having two separate patient interfaces as in FIG. 8. The surgical beam 3210 and the imaging beam 3220 are combined at the patient interface 3330 and are directed to the target 1001 by the common patient interface 3300. In addition, a common control module 3100 is provided to control both the imaging sub-system 2230 and the surgical part (the laser engine 2130 and the beam delivery system 2140). This increased integration between imaging and surgical parts allows accurate calibration of the two subsystems and the stability of the position of the patient and surgical volume. A common housing 3400 is provided to enclose both the surgical and imaging subsystems. When the two systems are not integrated into a common housing, the common patient interface 3300 can be part of either the imaging or the surgical subsystem.

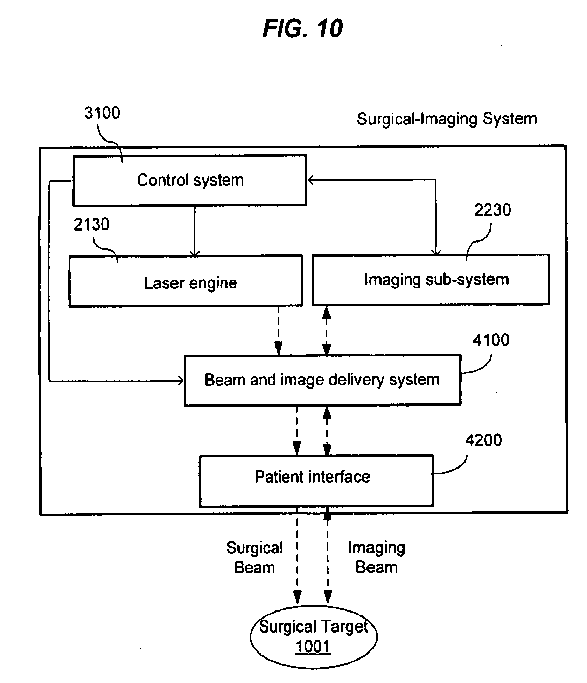

FIG. 10 shows an example of an imaging-guided laser surgical system where the laser surgical system and the imaging system share both a common beam delivery module 4100 and a common patient interface 4200. This integration further simplifies the system structure and system control operation.

In one implementation, the imaging system in the above and other examples can be an optical computed tomography (OCT) system and the laser surgical system is a femtosecond or picosecond laser based ophthalmic surgical system. In OCT, light from a low coherence, broadband light source such as a super luminescent diode is split into separate reference and signal beams. The signal beam is the imaging beam sent to the surgical target and the returned light of the imaging beam is collected and recombined coherently with the reference beam to form an interferometer. Scanning the signal beam perpendicularly to the optical axis of the optical train or the propagation direction of the light provides spatial resolution in the x-y direction while depth resolution comes from extracting differences between the path lengths of the reference arm and the returned signal beam in the signal arm of the interferometer. While the x-y scanner of different OCT implementations are essentially the same, comparing the path lengths and getting z-scan information can happen in different ways. In one implementation known as the time domain OCT, for example, the reference arm is continuously varied to change its path length while a photodetector detects interference modulation in the intensity of the re-combined beam. In a different implementation, the reference arm is essentially static and the spectrum of the combined light is analyzed for interference. The Fourier transform of the spectrum of the combined beam provides spatial information on the scattering from the interior of the sample. This method is known as the spectral domain or Fourier OCT method. In a different implementation known as a frequency swept OCT (S. R. Chinn, et. al., Opt. Lett. 22, 1997), a narrowband light source is used with its frequency swept rapidly across a spectral range. Interference between the reference and signal arms is detected by a fast detector and dynamic signal analyzer. An external cavity tuned diode laser or frequency tuned of frequency domain mode-locked (FDML) laser developed for this purpose (R. Huber et. Al. Opt. Express, 13, 2005) (S. H. Yun, IEEE J. of Sel. Q. El. 3(4) p. 1087-1096, 1997) can be used in these examples as a light source. A femtosecond laser used as a light source in an OCT system can have sufficient bandwidth and can provide additional benefits of increased signal to noise ratios.

The OCT imaging device in the systems in this document can be used to perform various imaging functions. For example, the OCT can be used to suppress complex conjugates resulting from the optical configuration of the system or the presence of the applanation plate, capture OCT images of selected locations inside the target tissue to provide three-dimensional positioning information for controlling focusing and scanning of the surgical laser beam inside the target tissue, or capture OCT images of selected locations on the surface of the target tissue or on the applanation plate to provide positioning registration for controlling changes in orientation that occur with positional changes of the target, such as from upright to supine. The OCT can be calibrated by a positioning registration process based on placement of marks or markers in one positional orientation of the target that can then be detected by the OCT module when the target is in another positional orientation. In other implementations, the OCT imaging system can be used to produce a probe light beam that is polarized to optically gather the information on the internal structure of the eye. The laser beam and the probe light beam may be polarized in different polarizations. The OCT can include a polarization control mechanism that controls the probe light used for said optical tomography to polarize in one polarization when traveling toward the eye and in a different polarization when traveling away from the eye. The polarization control mechanism can include, e.g., a wave-plate or a Faraday rotator.

The system in FIG. 10 is shown as a spectral OCT configuration and can be configured to share the focusing optics part of the beam delivery module between the surgical and the imaging systems. The main requirements for the optics are related to the operating wavelength, image quality, resolution, distortion etc. The laser surgical system can be a femtosecond laser system with a high numerical aperture system designed to achieve diffraction limited focal spot sizes, e.g., about 2 to 3 micrometers. Various femtosecond ophthalmic surgical lasers can operate at various wavelengths such as wavelengths of around 1.05 micrometer. The operating wavelength of the imaging device can be selected to be close to the laser wavelength so that the optics is chromatically compensated for both wavelengths. Such a system may include a third optical channel, a visual observation channel such as a surgical microscope, to provide an additional imaging device to capture images of the target tissue. If the optical path for this third optical channel shares optics with the surgical laser beam and the light of the OCT imaging device, the shared optics can be configured with chromatic compensation in the visible spectral band for the third optical channel and the spectral bands for the surgical laser beam and the OCT imaging beam.

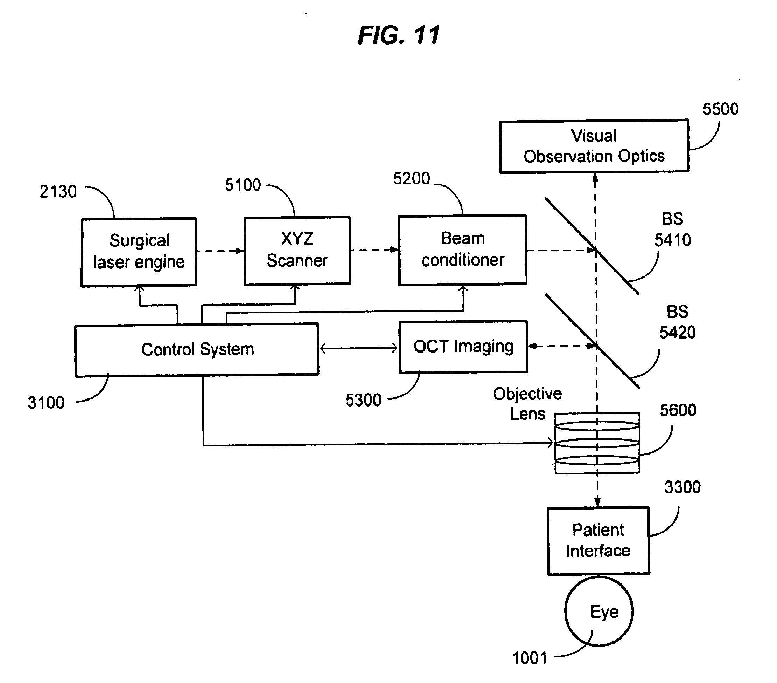

FIG. 11 shows a particular example of the design in FIG. 9 where the scanner 5100 for scanning the surgical laser beam and the beam conditioner 5200 for conditioning (collimating and focusing) the surgical laser beam are separate from the optics in the OCT imaging module 5300 for controlling the imaging beam for the OCT. The surgical and imaging systems share an objective lens 5600 module and the patient interface 3300. The objective lens 5600 directs and focuses both the surgical laser beam and the imaging beam to the patient interface 3300 and its focusing is controlled by the control module 3100. Two beam splitters 5410 and 5420 are provided to direct the surgical and imaging beams. The beam splitter 5420 is also used to direct the returned imaging beam back into the OCT imaging module 5300. Two beam splitters 5410 and 5420 also direct light from the target 1001 to a visual observation optics unit 5500 to provide direct view or image of the target 1001. The unit 5500 can be a lens imaging system for the surgeon to view the target 1001 or a camera to capture the image or video of the target 1001. Various beam splitters can be used, such as dichroic and polarization beam splitters, optical grating, holographic beam splitter or a combinations of these.

In some implementations, the optical components may be appropriately coated with antireflection coating for both the surgical and for the OCT wavelength to reduce glare from multiple surfaces of the optical beam path. Reflections would otherwise reduce the throughput of the system and reduce the signal to noise ratio by increasing background light in the OCT imaging unit. One way to reduce glare in the OCT is to rotate the polarization of the return light from the sample by wave-plate of Faraday isolator placed close to the target tissue and orient a polarizer in front of the OCT detector to preferentially detect light returned from the sample and suppress light scattered from the optical components.

In a laser surgical system, each of the surgical laser and the OCT system can have a beam scanner to cover the same surgical region in the target tissue. Hence, the beam scanning for the surgical laser beam and the beam scanning for the imaging beam can be integrated to share common scanning devices.

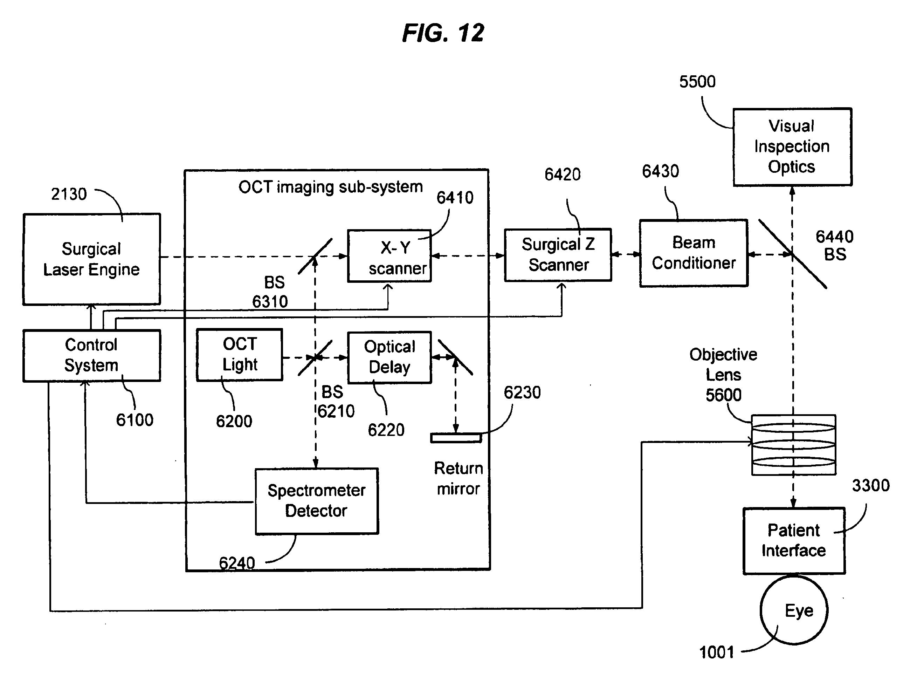

FIG. 12 shows an example of such a system in detail. In this implementation the x-y scanner 6410 and the z scanner 6420 are shared by both subsystems. A common control 6100 is provided to control the system operations for both surgical and imaging operations. The OCT sub-system includes an OCT light source 6200 that produce the imaging light that is split into an imaging beam and a reference beam by a beam splitter 6210. The imaging beam is combined with the surgical beam at the beam splitter 6310 to propagate along a common optical path leading to the target 1001. The scanners 6410 and 6420 and the beam conditioner unit 6430 are located downstream from the beam splitter 6310. A beam splitter 6440 is used to direct the imaging and surgical beams to the objective lens 5600 and the patient interface 3300.

In the OCT sub-system, the reference beam transmits through the beam splitter 6210 to an optical delay device 6220 and is reflected by a return mirror 6230. The returned imaging beam from the target 1001 is directed back to the beam splitter 6310 which reflects at least a portion of the returned imaging beam to the beam splitter 6210 where the reflected reference beam and the returned imaging beam overlap and interfere with each other. A spectrometer detector 6240 is used to detect the interference and to produce OCT images of the target 1001. The OCT image information is sent to the control system 6100 for controlling the surgical laser engine 2130, the scanners 6410 and 6420 and the objective lens 5600 to control the surgical laser beam. In one implementation, the optical delay device 6220 can be varied to change the optical delay to detect various depths in the target tissue 1001.

If the OCT system is a time domain system, the two subsystems use two different z-scanners because the two scanners operate in different ways. In this example, the z scanner of the surgical system operates by changing the divergence of the surgical beam in the beam conditioner unit without changing the path lengths of the beam in the surgical beam path. On the other hand, the time domain OCT scans the z-direction by physically changing the beam path by a variable delay or by moving the position of the reference beam return mirror. After calibration, the two z-scanners can be synchronized by the laser control module. The relationship between the two movements can be simplified to a linear or polynomial dependence, which the control module can handle or alternatively calibration points can define a look-up table to provide proper scaling. Spectral / Fourier domain and frequency swept source OCT devices have no z-scanner, the length of the reference arm is static. Besides reducing costs, cross calibration of the two systems will be relatively straightforward. There is no need to compensate for differences arising from image distortions in the focusing optics or from the differences of the scanners of the two systems since they are shared.

In practical implementations of the surgical systems, the focusing objective lens 5600 is slidably or movably mounted on a base and the weight of the objective lens is balanced to limit the force on the patient's eye. The patient interface 3300 can include an applanation lens attached to a patient interface mount. The patient interface mount is attached to a mounting unit, which holds the focusing objective lens. This mounting unit is designed to ensure a stable connection between the patient interface and the system in case of unavoidable movement of the patient and allows gentler docking of the patient interface onto the eye. Various implementations for the focusing objective lens can be used and one example is described in U.S. Patent 5,336,215 to Hsueh. This presence of an adjustable focusing objective lens can change the optical path length of the optical probe light as part of the optical interferometer for the OCT sub-system. Movement of the objective lens 5600 and patient interface 3300 can change the path length differences between the reference beam and the imaging signal beam of the OCT in an uncontrolled way and this may degrade the OCT depth information detected by the OCT. This would happen not only in time-domain but also in spectral / Fourier domain and frequency-swept OCT systems.

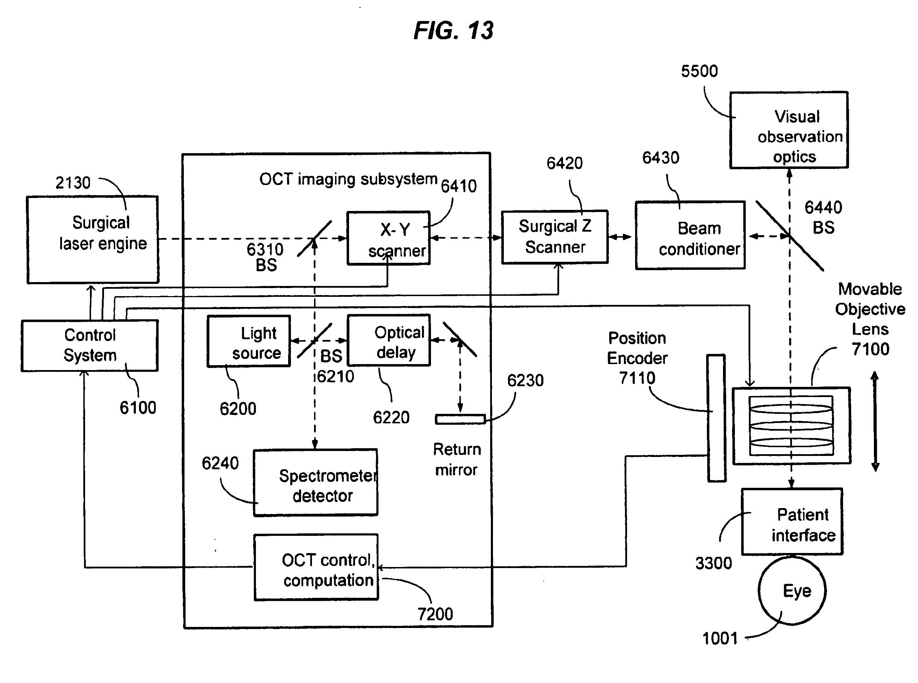

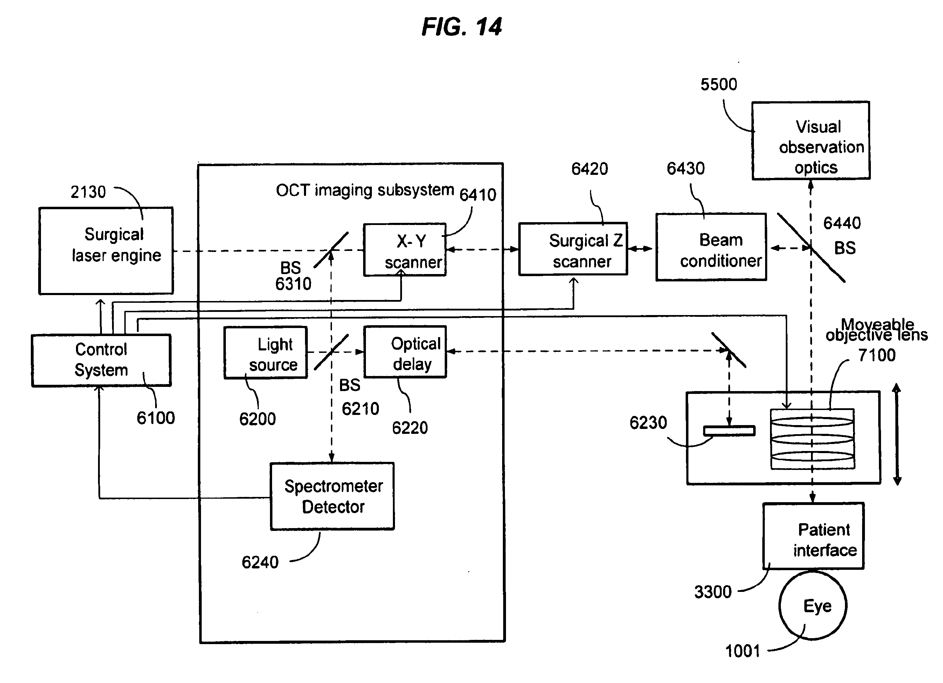

FIGs. 13 and 14 show exemplary imaging-guided laser surgical systems that address the technical issue associated with the adjustable focusing objective lens.

The system in FIG. 13 provides a position sensing device 7110 coupled to the movable focusing objective lens 7100 to measure the position of the objective lens 7100 on a slideable mount and communicates the measured position to a control module 7200 in the OCT system. The control system 6100 can control and move the position of the objective lens 7100 to adjust the optical path length traveled by the imaging signal beam for the OCT operation and the position of the lens 7100 is measured and monitored by the position encoder 7110 and direct fed to the OCT control 7200. The control module 7200 in the OCT system applies an algorithm, when assembling a 3D image in processing the OCT data, to compensate for differences between the reference arm and the signal arm of the interferometer inside the OCT caused by the movement of the focusing objective lens 7100 relative to the patient interface 3300. The proper amount of the change in the position of the lens 7100 computed by the OCT control module 7200 is sent to the control 6100 which controls the lens 7100 to change its position.

FIG. 14 shows another exemplary system where the return mirror 6230 in the reference arm of the interferometer of the OCT system or at least one part in an optical path length delay assembly of the OCT system is rigidly attached to the movable focusing objective lens 7100 so the signal arm and the reference arm undergo the same amount of change in the optical path length when the objective lens 7100 moves. As such, the movement of the objective lens 7100 on the slide is automatically compensated for pathlength differences in the OCT system without additional need for a computational compensation.

The above examples for imaging-guided laser surgical systems, the laser surgical system and the OCT system use different light sources. In an even more complete integration between the laser surgical system and the OCT system, a femtosecond surgical laser as a light source for the surgical laser beam can also be used as the light source for the OCT system.

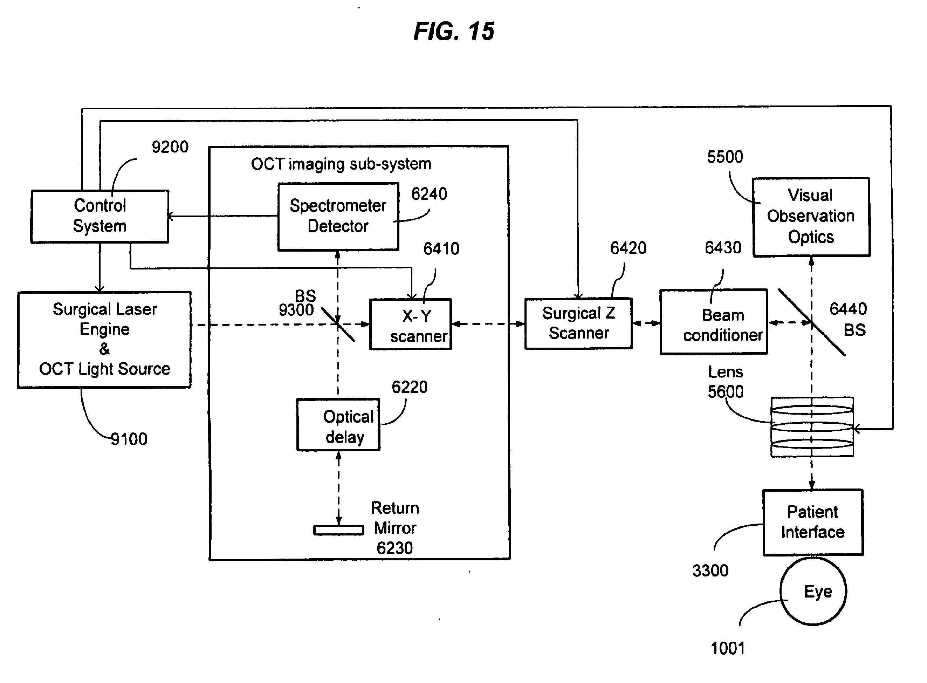

FIG. 15 shows an example where a femtosecond pulse laser in a light module 9100 is used to generate both the surgical laser beam for surgical operations and the probe light beam for OCT imaging. A beam splitter 9300 is provided to split the laser beam into a first beam as both the surgical laser beam and the signal beam for the OCT and a second beam as the reference beam for the OCT. The first beam is directed through an x-y scanner 6410 which scans the beam in the x and y directions perpendicular to the propagation direction of the first beam and a second scanner (z scanner) 6420 that changes the divergence of the beam to adjust the focusing of the first beam at the target tissue 1001. This first beam performs the surgical operations at the target tissue 1001 and a portion of this first beam is back scattered to the patient interface and is collected by the objective lens as the signal beam for the signal arm of the optical interferometer of the OCT system. This returned light is combined with the second beam that is reflected by a return mirror 6230 in the reference arm and is delayed by an adjustable optical delay element 6220 for a time-domain OCT to control the path difference between the signal and reference beams in imaging different depths of the target tissue 1001. The control system 9200 controls the system operations.

Surgical practice on the cornea has shown that a pulse duration of several hundred femtoseconds may be sufficient to achieve good surgical performance, while for OCT of a sufficient depth resolution broader spectral bandwidth generated by shorter pulses, e.g., below several tens of femtoseconds, are needed. In this context, the design of the OCT device dictates the duration of the pulses from the femtosecond surgical laser.

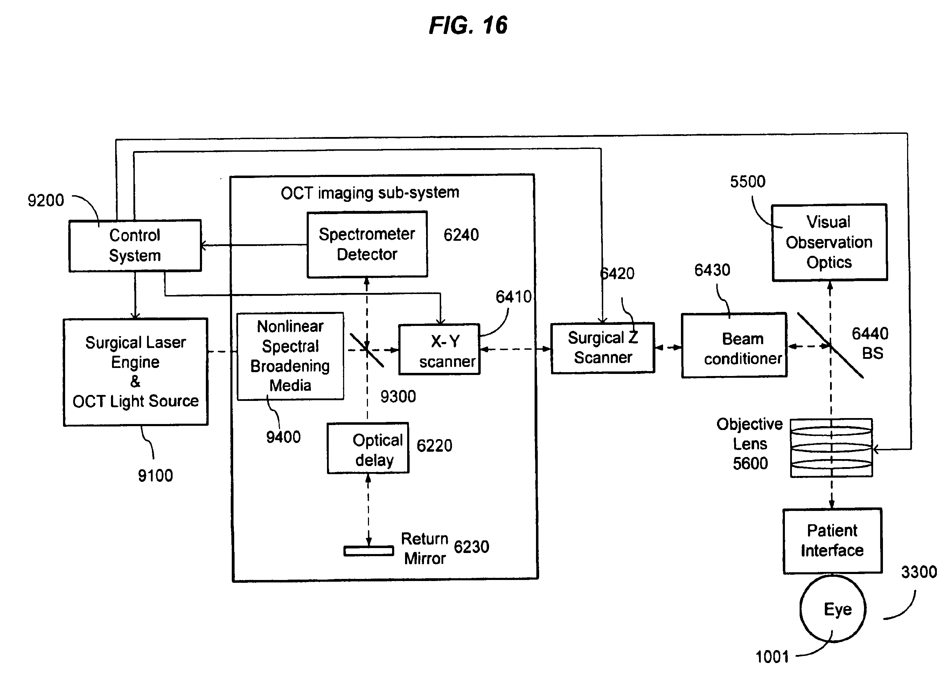

FIG. 16 shows another imaging-guided system that uses a single pulsed laser 9100 to produce the surgical light and the imaging light. A nonlinear spectral broadening media 9400 is placed in the output optical path of the femtosecond pulsed laser to use an optical non-linear process such as white light generation or spectral broadening to broaden the spectral bandwidth of the pulses from a laser source of relatively longer pulses, several hundred femtoseconds normally used in surgery. The media 9400 can be a fiber-optic material, for example. The light intensity requirements of the two systems are different and a mechanism to adjust beam intensities can be implemented to meet such requirements in the two systems. For example, beam steering mirrors, beam shutters or attenuators can be provided in the optical paths of the two systems to properly control the presence and intensity of the beam when taking an OCT image or performing surgery in order to protect the patient and sensitive instruments from excessive light intensity.

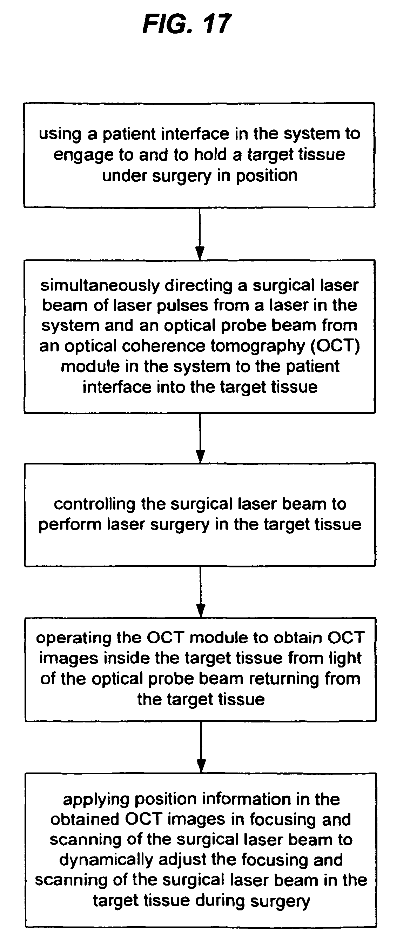

In operation, the above examples in FIGs. 8/16 can be used to perform imaging-guided laser surgery. FIG. 17 shows one example of a method for performing laser surgery by using an imaging-guided laser surgical system. This method uses a patient interface in the system to engage to and to hold a target tissue under surgery in position and simultaneously directs a surgical laser beam of laser pulses from a laser in the system and an optical probe beam from the OCT module in the system to the patient interface into the target tissue. The surgical laser beam is controlled to perform laser surgery in the target tissue and the OCT module is operated to obtain OCT images inside the target tissue from light of the optical probe beam returning from the target tissue. The position information in the obtained OCT images is applied in focusing and scanning of the surgical laser beam to adjust the focusing and scanning of the surgical laser beam in the target tissue before or during surgery.

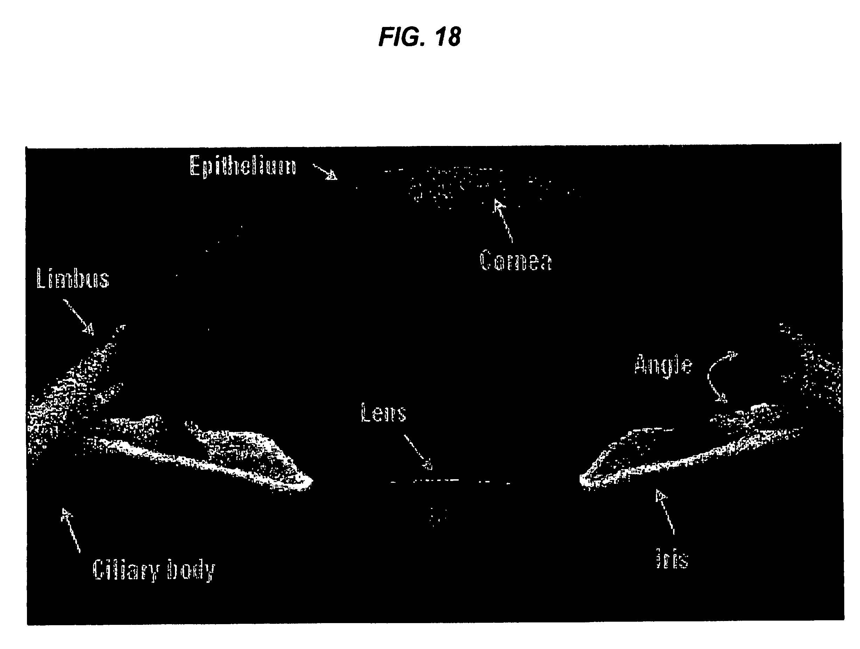

FIG. 18 shows an example of an OCT image of an eye. The contacting surface of the applanation lens in the patent interface can be configured to have a curvature that minimizes distortions or folds in the cornea due to the pressure exerted on the eye during applanation. After the eye is successfully applanated at the patient interface, an OCT image can be obtained. As shown in FIG. 18, the curvature of the lens and cornea as well as the distances between the lens and cornea are identifiable in the OCT image. Subtler features such as the epithelium-cornea interface are detectable. Each of these identifiable features may be used as an internal reference of the laser coordinates with the eye. The coordinates of the cornea and lens can be digitized using well-established computer vision algorithms such as Edge or Blob detection. Once the coordinates of the lens are established, they can be used to control the focusing and positioning of the surgical laser beam for the surgery.

Alternatively, a calibration sample material may be used to form a 3-D array of reference marks at locations with known position coordinates. The OCT image of the calibration sample material can be obtained to establish a mapping relationship between the known position coordinates of the reference marks and the OCT images of the reference marks in the obtained OCT image. This mapping relationship is stored as digital calibration data and is applied in controlling the focusing and scanning of the surgical laser beam during the surgery in the target tissue based on the OCT images of the target tissue obtained during the surgery. The OCT imaging system is used here as an example and this calibration can be applied to images obtained via other imaging techniques.

In an imaging-guided laser surgical system described here, the surgical laser can produce relatively high peak powers sufficient to drive strong field/multi-photon ionization inside of the eye (i.e. inside of the cornea and lens) under high numerical aperture focusing. Under these conditions, one pulse from the surgical laser generates a plasma within the focal volume. Cooling of the plasma results in a well defined damage zone or "bubble" that may be used as a reference point. The following sections describe a calibration procedure for calibrating the surgical laser against an OCT-based imaging system using the damage zones created by the surgical laser.

Before surgery can be performed, the OCT is calibrated against the surgical laser to establish a relative positioning relationship so that the surgical laser can be controlled in position at the target tissue with respect to the position associated with images in the OCT image of the target tissue obtained by the OCT. One way for performing this calibration uses a pre-calibrated target or "phantom" which can be damaged by the laser as well as imaged with the OCT. The phantom can be fabricated from various materials such as a glass or hard plastic (e.g. PMMA) such that the material can permanently record optical damage created by the surgical laser. The phantom can also be selected to have optical or other properties (such as water content) that are similar to the surgical target.

The phantom can be, e.g., a cylindrical material having a diameter of at least 10 mm (or that of the scanning range of the delivery system) and a cylindrical length of at least 10 mm long spanning the distance of the epithelium to the crystalline lens of the eye, or as long as the scanning depth of the surgical system. The upper surface of the phantom can be curved to mate seamlessly with the patient interface or the phantom material may be compressible to allow full applanation. The phantom may have a three dimensional grid such that both the laser position (in x and y) and focus (z), as well as the OCT image can be referenced against the phantom.

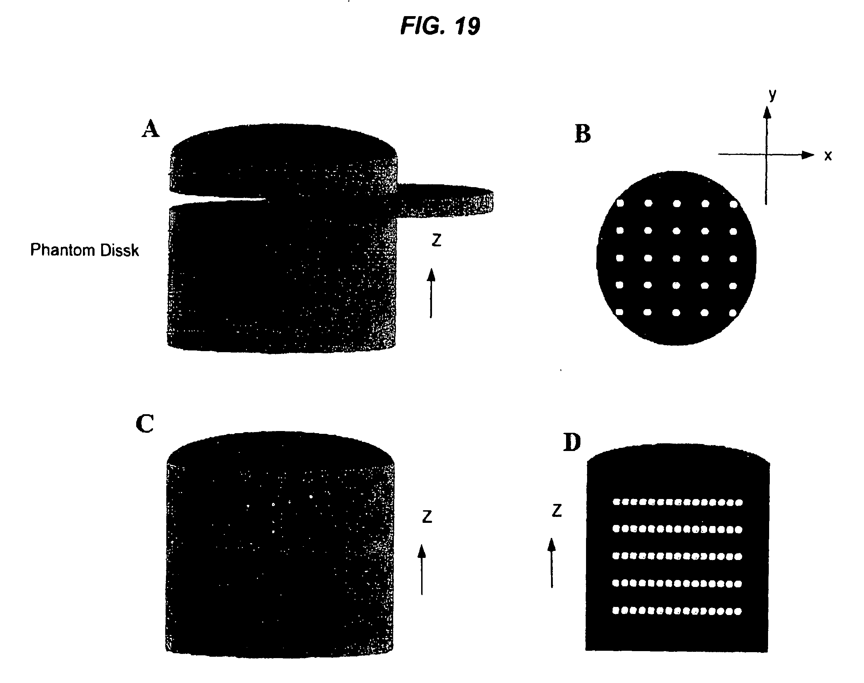

FIGs. 19A-19D show two exemplary configurations for the phantom. FIG. 19A shows a phantom that is segmented into thin disks. FIG. 19B shows a single disk patterned to have a grid of reference marks as a reference for determining the laser position across the phantom (i.e. the x- and y- coordinates). The z-coordinate (depth) can be determined by removing an individual disk from the stack and imaging it under a confocal microscope. FIG. 19C shows a phantom that can be separated into two halves. Similar to the segmented phantom in FIG. 19A, this phantom is structured to contain a grid of reference marks as a reference for determining the laser position in the x- and y- coordinates. Depth information can be extracted by separating the phantom into the two halves and measuring the distance between damage zones. The combined information can provide the parameters for image guided surgery.

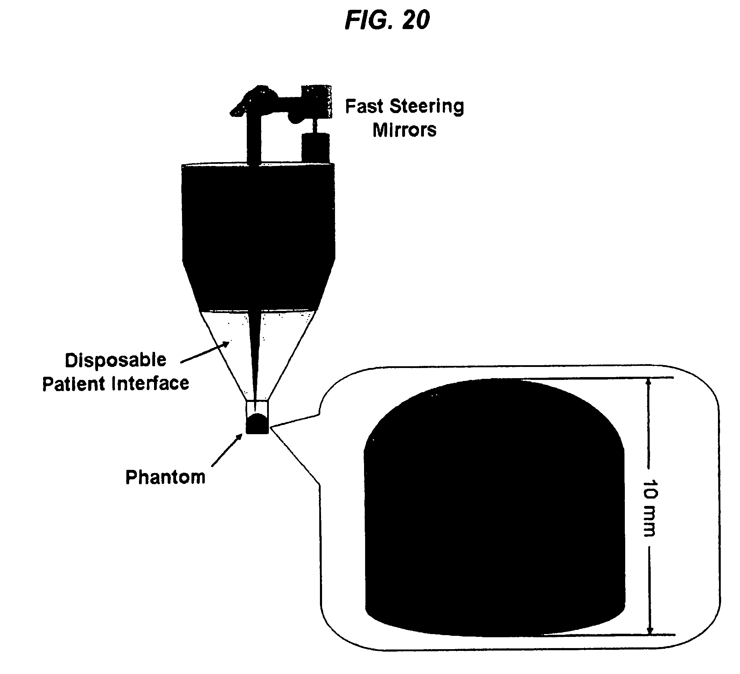

FIG. 20 shows a surgical system part of the imaging-guided laser surgical system. This system includes steering mirrors which may be actuated by actuators such as galvanometers or voice coils, an objective lens e and a disposable patient interface. The surgical laser beam is reflected from the steering mirrors through the objective lens. The objective lens focuses the beam just after the patient interface. Scanning in the x- and y-coordinates is performed by changing the angle of the beam relative to the objective lens. Scanning in z-plane is accomplished by changing the divergence of the incoming beam using a system of lens upstream to the steering mirrors.

In this example, the conical section of the disposable patient interface may be either air spaced or solid and the section interfacing with the patient includes a curved contact lens. The curved contact lens can be fabricated from fused silica or other material resistant to forming color centers when irradiated with ionizing radiation. The radius of curvature is on the upper limit of what is compatible with the eye, e.g., about 10 mm.

The first step in the calibration procedure is docking the patient interface with the phantom. The curvature of the phantom matches the curvature of the patient interface. After docking, the next step in the procedure involves creating optical damage inside of the phantom to produce the reference marks.

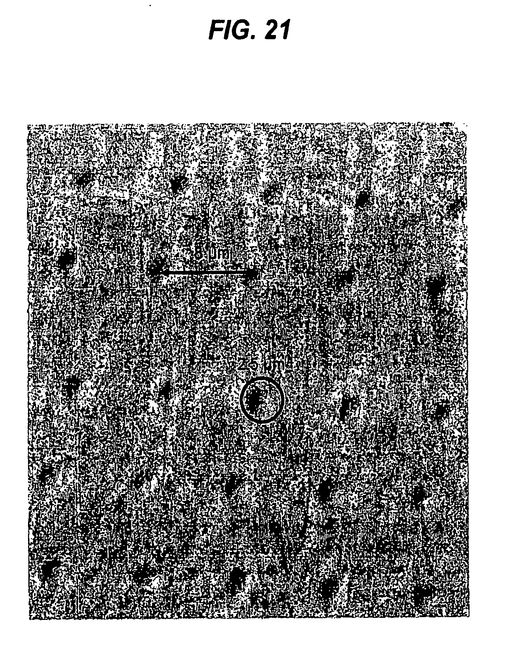

FIG. 21 shows examples of actual damage zones produced by a femtosecond laser in glass. The separation between the damage zones is on average 8 µm (the pulse energy is 2.2 µJ with duration of 580 fs at full width at half maximum). The optical damage depicted in FIG. 21 shows that the damage zones created by the femtosecond laser are well-defined and discrete. In the example shown, the damage zones have a diameter of about 2.5 µm. Optical damage zones similar to that shown in FIG. 20 are created in the phantom at various depths to form a 3-D array of the reference marks. These damage zones are referenced against the calibrated phantom either by extracting the appropriate disks and imaging it under a confocal microscope (FIG. 19A) or by splitting the phantom into two halves and measuring the depth using a micrometer (FIG. 19C). The x- and y- coordinates can be established from the pre-calibrated grid.

After damaging the phantom with the surgical laser, OCT on the phantom is performed. The OCT imaging system provides a 3D rendering of the phantom establishing a relationship between the OCT coordinate system and the phantom. The damage zones are detectable with the imaging system. The OCT and laser may be cross-calibrated using the phantom's internal standard. After the OCT and the laser are referenced against each other, the phantom can be discarded.

Prior to surgery, the calibration can be verified. This verification step involves creating optical damage at various positions inside of a second phantom. The optical damage should be intense enough such that the multiple damage zones which create a circular pattern can be imaged by the OCT. After the pattern is created, the second phantom is imaged with the OCT. Comparison of the OCT image with the laser coordinates provides the final check of the system calibration prior to surgery.

Once the coordinates are fed into the laser, laser surgery can be performed inside the eye. This involves photo-emulsification of the lens using the laser, as well as other laser treatments to the eye. The surgery can be stopped at any time and the anterior segment of the eye (FIG. 17) can be re-imaged to monitor the progress of the surgery; moreover, after the IOL (intra ocular lens) is inserted, imaging the IOL (with light or no applanation) provides information regarding the position of the IOL in the eye. This information may be utilized by the physician to refine the position of the IOL.

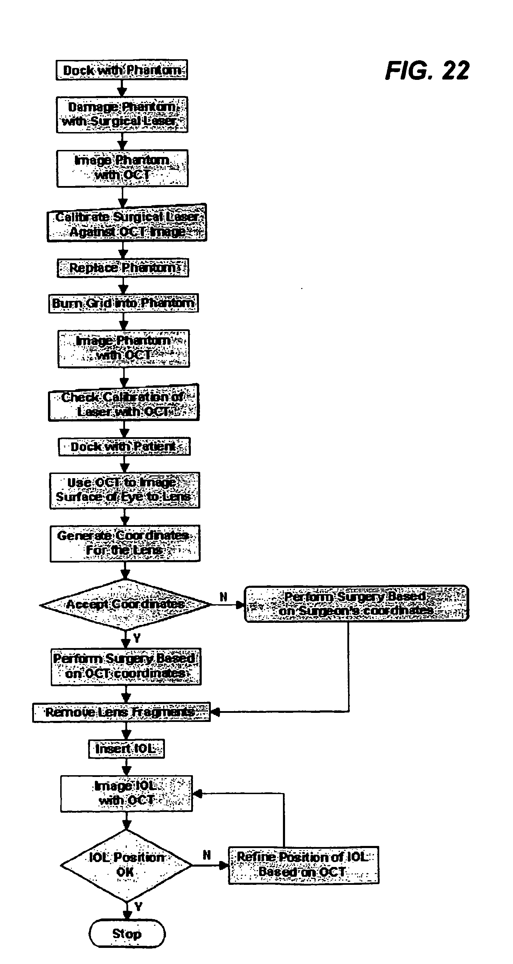

FIG. 22 shows an example of the calibration process and the post-calibration surgical operation. This example shows a method for performing laser surgery by using an imaging-guided laser surgical system can include using a patient interface in the system, that is engaged to hold a target tissue under surgery in position, to hold a calibration sample material during a calibration process before performing a surgery; directing a surgical laser beam of laser pulses from a laser in the system to the patient interface into the calibration sample material to burn reference marks at selected three-dimensional reference locations; directing an optical probe beam from an optical coherence tomography (OCT) module in the system to the patient interface into the calibration sample material to capture OCT images of the burnt reference marks; and establishing a relationship between positioning coordinates of the OCT module and the burnt reference marks. After the establishing the relationship, a patient interface in the system is used to engage to and to hold a target tissue under surgery in position. The surgical laser beam of laser pulses and the optical probe beam are directed to the patient interface into the target tissue. The surgical laser beam is controlled to perform laser surgery in the target tissue. The OCT module is operated to obtain OCT images inside the target tissue from light of the optical probe beam returning from the target tissue and the position information in the obtained OCT images and the established relationship are applied in focusing and scanning of the surgical laser beam to adjust the focusing and scanning of the surgical laser beam in the target tissue during surgery. While such calibrations can be performed immediately prior to laser surgery, they can also be performed at various intervals before a procedure, using calibration validations that demonstrated a lack of drift or change in calibration during such intervals.

The following examples describe imaging-guided laser surgical techniques and systems that use images of laser-induced photodisruption byproducts for alignment of the surgical laser beam.

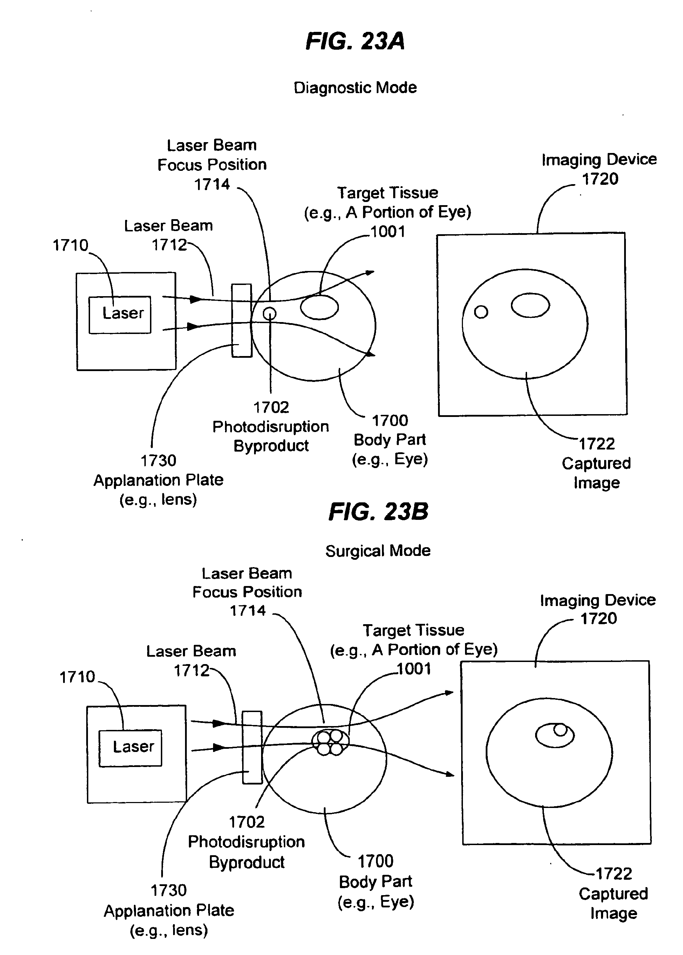

FIGs. 23A and 23B show another implementation of the present technique in which actual photodisruption byproducts in the target tissue are used to guide further laser placement. A pulsed laser 1710, such as a femtosecond or picosecond laser, is used to produce a laser beam 1712 with laser pulses to cause photodisruption in a target tissue 1001. The target tissue 1001 may be a part of a body part 1700 of a subject, e.g., a portion of the lens of one eye. The laser beam 1712 is focused and directed by an optics module for the laser 1710 to a target tissue position in the target tissue 1001 to achieve a certain surgical effect. The target surface is optically coupled to the laser optics module by an applanation plate 1730 that transmits the laser wavelength, as well as image wavelengths from the target tissue. The applanation plate 1730 can be an applanation lens. An imaging device 1720 is provided to collect reflected or scattered light or sound from the target tissue 1001 to capture images of the target tissue 1001 either before or after (or both) the applanation plate is applied. The captured imaging data is then processed by the laser system control module to determine the desired target tissue position. The laser system control module moves or adjusts optical or laser elements based on standard optical models to ensure that the center of photodisruption byproduct 1702 overlaps with the target tissue position. This can be a dynamic alignment process where the images of the photodisruption byproduct 1702 and the target tissue 1001 are continuously monitored during the surgical process to ensure that the laser beam is properly positioned at each target tissue position.

In one implementation, the laser system can be operated in two modes: first in a diagnostic mode in which the laser beam 1712 is initially aligned by using alignment laser pulses to create photodisruption byproduct 1702 for alignment and then in a surgical mode where surgical laser pulses are generated to perform the actual surgical operation. In both modes, the images of the disruption byproduct 1702 and the target tissue 1001 are monitored to control the beam alignment. FIG. 17A shows the diagnostic mode where the alignment laser pulses in the laser beam 1712 may be set at a different energy level than the energy level of the surgical laser pulses. For example, the alignment laser pulses may be less energetic than the surgical laser pulses but sufficient to cause significant photodisruption in the tissue to capture the photodisruption byproduct 1702 at the imaging device 1720. The resolution of this coarse targeting may not be sufficient to provide desired surgical effect. Based on the captured images, the laser beam 1712 can be aligned properly. After this initial alignment, the laser 1710 can be controlled to produce the surgical laser pulses at a higher energy level to perform the surgery. Because the surgical laser pulses are at a different energy level than the alignment laser pulses, the nonlinear effects in the tissue material in the photodisruption can cause the laser beam 1712 to be focused at a different position from the beam position during the diagnostic mode. Therefore, the alignment achieved during the diagnostic mode is a coarse alignment and additional alignment can be further performed to precisely position each surgical laser pulse during the surgical mode when the surgical laser pulses perform the actual surgery. Referring to FIG. 23A, the imaging device 1720 captures the images from the target tissue 1001 during the surgical mode and the laser control module adjust the laser beam 1712 to place the focus position 1714 of the laser beam 1712 onto the desired target tissue position in the target tissue 1001. This process is performed for each target tissue position.

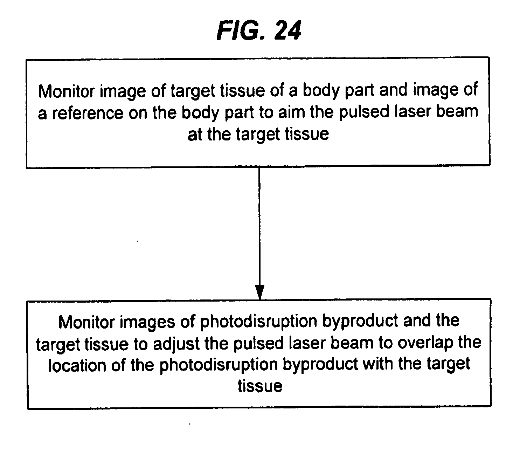

FIG. 24 shows one implementation of the laser alignment where the laser beam is first approximately aimed at the target tissue and then the image of the photodisruption byproduct is captured and used to align the laser beam. The image of the target tissue of the body part as the target tissue and the image of a reference on the body part are monitored to aim the pulsed laser beam at the target tissue. The images of photodisruption byproduct and the target tissue are used to adjust the pulsed laser beam to overlap the location of the photodisruption byproduct with the target tissue.

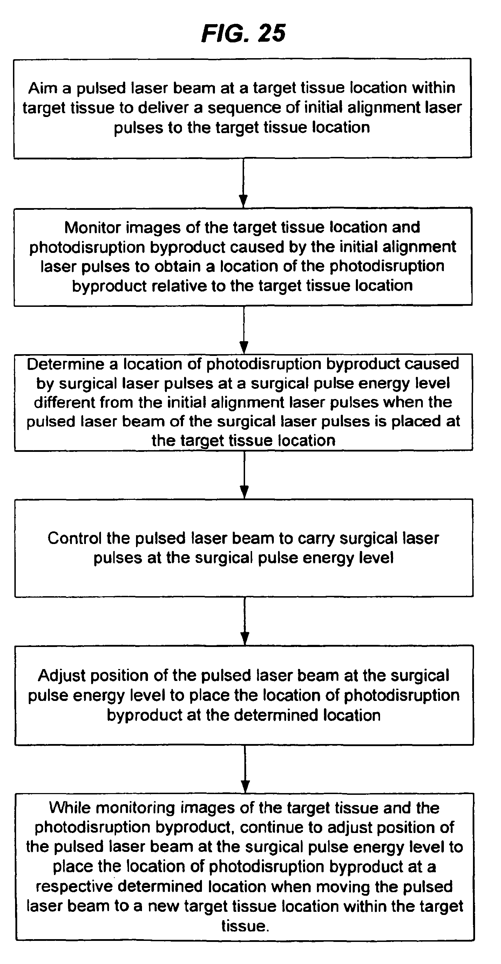

FIG. 25 shows one exemplary implementation of the laser alignment method based on imaging photodisruption byproduct in the target tissue in laser surgery. In this method, a pulsed laser beam is aimed at a target tissue location within target tissue to deliver a sequence of initial alignment laser pulses to the target tissue location. The images of the target tissue location and photodisruption byproduct caused by the initial alignment laser pulses are monitored to obtain a location of the photodisruption byproduct relative to the target tissue location. The location of photodisruption by product caused by surgical laser pulses at a surgical pulse energy level different from the initial alignment laser pulses is determined when the pulsed laser beam of the surgical laser pulses is placed at the target tissue location. The pulsed laser beam is controlled to carry surgical laser pulses at the surgical pulse energy level. The position of the pulsed laser beam is adjusted at the surgical pulse energy level to place the location of photodisruption byproduct at the determined location. While monitoring images of the target tissue and the photodisruption byproduct, the position of the pulsed laser beam at the surgical pulse energy level is adjusted to place the location of photodisruption byproduct at a respective determined location when moving the pulsed laser beam to a new target tissue location within the target tissue.

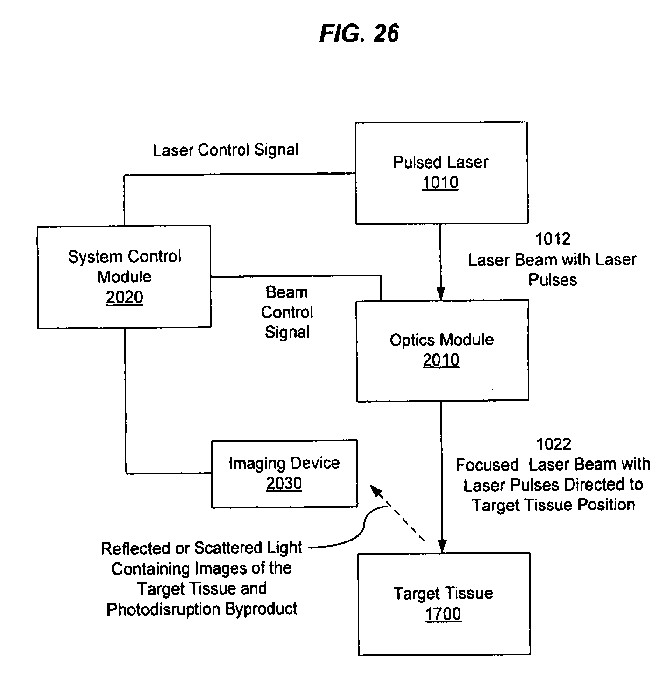

FIG. 26 shows an exemplary laser surgical system based on the laser alignment using the image of the photodisruption byproduct. An optics module 2010 is provided to focus and direct the laser beam to the target tissue 1700. The optics module 2010 can include one or more lenses and may further include one or more reflectors. A control actuator is included in the optics module 2010 to adjust the focusing and the beam direction in response to a beam control signal. A system control module 2020 is provided to control both the pulsed laser 1010 via a laser control signal and the optics module 2010 via the beam control signal. The system control module 2020 processes image data from the imaging device 2030 that includes the position offset information for the photodisruption byproduct 1702 from the target tissue position in the target tissue 1700. Based on the information obtained from the image, the beam control signal is generated to control the optics module 2010 which adjusts the laser beam. A digital processing unit is included in the system control module 2020 to perform various data processing for the laser alignment.

The imaging device 2030 can be implemented in various forms, including an optical coherent tomography (OCT) device. In addition, an ultrasound imaging device can also be used. The position of the laser focus is moved so as to place it grossly located at the target at the resolution of the imaging device. The error in the referencing of the laser focus to the target and possible non-linear optical effects such as self focusing that make it difficult to accurately predict the location of the laser focus and subsequent photodisruption event. Various calibration methods, including the use of a model system or software program to predict focusing of the laser inside a material can be used to get a coarse targeting of the laser within the imaged tissue. The imaging of the target can be performed both before and after the photodisruption. The position of the photodisruption by products relative to the target is used to shift the focal point of the laser to better localize the laser focus and photodisruption process at or relative to the target. Thus the actual photodisruption event is used to provide a precise targeting for the placement of subsequent surgical pulses.

Photodisruption for targeting during the diagnostic mode can be performed at a lower, higher or the same energy level that is required for the later surgical processing in the surgical mode of the system. A calibration may be used to correlate the localization of the photodisruptive event performed at a different energy in diagnostic mode with the predicted localization at the surgical energy because the optical pulse energy level can affect the exact location of the photodisruptive event. Once this initial localization and alignment is performed, a volume or pattern of laser pulses (or a single pulse) can be delivered relative to this positioning. Additional sampling images can be made during the course of delivering the additional laser pulses to ensure proper localization of the laser (the sampling images may be obtained with use of lower, higher or the same energy pulses). In one implementation, an ultrasound device is used to detect the cavitation bubble or shock wave or other photodisruption byproduct. The localization of this can then be correlated with imaging of the target, obtained via ultrasound or other modality. The imaging device may be simply a biomicroscope or other optical visualization of the photodisruption event by the operator, such as optical coherence tomography. With the initial observation, the laser focus is moved to the desired target position, after which a pattern or volume of pulses is delivered relative to this initial position.

As a specific example, a laser system for precise subsurface photodisruption can include means for generating laser pulses capable of generating photodisruption at repetition rates of 100-1000 Million pulses per second, means for coarsely focusing laser pulses to a target below a surface using an image of the target and a calibration of the laser focus to that image without creating a surgical effect, means for detecting or visualizing below a surface to provide an image or visualization of a target the adjacent space or material around the target and the byproducts of at least one photodisruptive event coarsely localized near the target, means for correlating the position of the byproducts of photodisruption with that of the sub surface target at least once and moving the focus of the laser pulse to position the byproducts of photodisruption at the sub surface target or at a relative position relative to the target, means for delivering a subsequent train of at least one additional laser pulse in pattern relative to the position indicated by the above fine correlation of the byproducts of photodisruption with that of the sub surface target, and means for continuing to monitor the photodisruptive events during placement of the subsequent train of pulses to further fine tune the position of the subsequent laser pulses relative to the same or revised target being imaged.

The above techniques and systems can be used deliver high repetition rate laser pulses to subsurface targets with a precision required for contiguous pulse placement, as needed for cutting or volume disruption applications. This can be accomplished with or without the use of a reference source on the surface of the target and can take into account movement of the target following applanation or during placement of laser pulses.

A few embodiments have been described in detail above, and various modifications are possible. The disclosed subject matter, including the functional operations described in this specification, can be implemented in electronic circuitry, computer hardware, firmware, software, or in combinations of them, such as the structural means disclosed in this specification and structural equivalents thereof, including potentially a program operable to cause one or more data processing apparatus to perform the operations described (such as a program encoded in a computer-readable medium, which can be a memory device, a storage device, a machine-readable storage substrate, or other physical, machine-readable medium, or a combination of one or more of them).

The term "data processing apparatus" encompasses all apparatus, devices, and machines the computer program in question, e.g., code that constitutes processor firmware, a protocol stack, a database management system, an operating system, or a combination of one or more of them.

A program (also known as a computer program, software, software application, script, or module, component, subroutine, or other unit suitable for use in a computing environment. A program does not necessarily correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A program can be deployed to be executed on one computer or on multiple computers that are located at one site or distributed across multiple sites and interconnected by a communication network.

While this specification contains many specifics, these should not be construed as limitation also be implemented in combination in a single embodiment. Conversely, various features that are described in the context of a single embodiment can also be implemented in multiple embodiments separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

Similarly, while operations are depicted in the drawings in a particular order, this should multitasking and parallel processing may be advantageous. Moreover, the separation of various system components in the embodiments described above should not be understood as requiring such separation in all embodiments.

Other embodiments fall within the scope of the following claims.

## Claims

1. A laser system for eye surgery, comprising:
a pulsed laser (1010, 9100, 1710) adapted to produce a beam of laser pulses (1012, 1022);
a mechanism adapted to align an eye along a selected axis (330) of the eye;
an imaging module (1030, 2030, 2200, 2230, 3400, 5300) adapted to monitor at least one of positioning of a physical reference mark (350-1, 350-2) made on or in the eye, and a selected reference image of at least one part of the eye relative to the selected axis of the eye; and
a control module (1040, 2020, 2120, 2220, 3100, 6100, 9200) in communication with the imaging module adapted to direct the laser pulses to at least one target (1001, 1700) in the eye relative to the selected axis of the eye,
wherein the laser system is configured
- to select an axis on which to align an optically impactful procedure;
- to make the physical reference mark on an external region of the eye to represent a position of the selected axis using a laser;
- to identify the physical reference mark and to determine the identified physical reference mark on an image of the eye; and
- to use a position of the identified physical reference mark on the image of the eye to direct laser pulses to at least one target in the eye relative to the physical reference mark or the selected axis of the eye
**characterized in that** said system is further adapted to place the physical reference mark on or in the eye when the eye is in a non-applanated state and to determine the reference image of the at least one part of the eye when the eye is in an applanted state.

2. The laser system of claim 1, further configured to use physical reference marks (350-1, 350-2) generated by laser pulses to guide a subsequent alignment.

3. The laser system of claim 1 or 2, further configured to align corneal incisions (360) and ablations.

4. The laser system of one of the preceding claims, further configured to use the physical reference marks to guide alignment or placement of intraocular or intra-corneal implant.

5. The laser system of one of the preceding claim, further configured to use information about the identified physical reference mark to assist a predicting of a position of an intraocular lens to be implanted in the eye.

6. The laser system of one of the preceding claims, wherein: the mechanism to align the eye is configured to fix a position of the eye relative to the selected axis.

7. The laser system of one of the preceding claims wherein: the imaging module is configured to monitor a position of the physical reference mark and a position of the reference image.

8. The laser system of one of the preceding claims, wherein at least one of the pulsed laser, the imaging module and the control module is configured to use mark on an intraocular lens or corneal inlay in conjunction with the physical reference mark to improve one of a positioning a choice of an optical power or size of the intraocular lens or corneal inlay.

9. The laser system of one of the preceding claims, comprising: a computer system to calculate a recommended intraocular lens power based at least in part on the position of the physical reference mark.

## Patentansprüche

1. Lagersystem für eine Augenoperation, aufweisend:
einen gepulsten Laser (1010, 9100,1710), der zur Erzeugung eines Strahls von Laserpulsen (1012, 1022) bestimmt ist;
ein Mechanismus, der dazu bestimmt ist, ein Auge entlang einer ausgewählten Achse (330) des Auges auszurichten;
ein Bildgeber-Modul (1030, 2030, 2200, 2230, 3400, 5300), das dazu bestimmt ist, mindestens eine Positionierung einer physikalischen Bezugsmarkierung 350-1, 350-2) zu überwachten, die auf oder in dem Auge erzeugt wurde, sowie ein ausgewähltes Bezugs-Bild von mindestens einem Teil des Auges relative zu der ausgewählten Achse des Auges; und
ein Steuerungs-Modul (1040, 2020, 2120, 2220, 3100, 6100, 9200) in Kommunikation mit dem Bildgeber-Modul, das dazu bestimmt ist, die Laserpulse auf mindestens ein Target (1001, 1700) in dem Auge relative zu der ausgewählten Achse des Auges auszurichten,
wobei das Lasersystem dazu bestimmt ist,
- eine Achse auszuwählen, auf der es ein optisch wirkungsvolles Prozedere auszurichten gilt;
- die physikalische Bezugsmarkierung auf eine externe Region des Auges zur Repräsentation einer Position der ausgewählten Achse unter Verwendung eines Lasers heranzuziehen;
- die physikalische Bezugsmarkierung zu identifizieren und die identifizierte physikalische Bezugsmarkierung auf einem Bild des Auges zu bestimmen; und
- eine Position der identifizierten physikalischen Bezugsmarkierung auf dem Bild des Auges zu verwenden, um Laserpulse auf mindestens ein Target in dem Auge relativ zu der physikalischen Bezugsmarkierung oder der ausgewählten Achse des Auges auszurichten,
**dadurch gekennzeichnet, dass** system ferner dazu bestimmt ist, die physikalische Bezugsmarkierung auf oder in dem Auge zu platzieren, wenn das Auge in einem nicht-applanierten Zustand ist, und um das Bezugsbild des mindestens einen Teils des Auges zu bestimmen, wenn sich das Auge in einem applanierten Zustand befindet.

2. Lasersystem nach Anspruch 1, das ferner konfiguriert ist, um physikalische Bezugsmarkierungen (350-1, 350-2) zu verwenden, die durch Laserpulse generiert sind, um eine darauffolgende Ausrichtung zu führen.

3. Lasersystem nach Anspruch 1 oder 2, ferner dazu konfiguriert, korneale Einschnitte (360) und Ablationen auszurichten.

4. Lagersystem nach einem der vorangehenden Ansprüche, feiner konfiguriert zur Verwendung der physikalischen Bezugsmarkierungen, um eine Ausrichtung oder Platzierung eines intraokularen oder intrakornealen Implantates zu führen.

5. Lasersystem nach einem der vorangehenden Ansprüche, ferner konfiguriert, um Informationen über die identifizierte physikalische Bezugsmarkierung zu verwenden, um eine Vorbestimmung einer Position einer intraokularen Linse zu unterstützen, die in dem Auge zu implantieren ist.

6. Lasersystem nach einem der vorangehenden Ansprüche, wobei der Mechanismus zur Ausrichtung des Auges dazu konfiguriert ist, eine Position des Auges relativ zu der ausgewählten Achse zu fixieren.

7. Lagersystem nach einem der vorangehenden Ansprüche, wobei das Bildgebermodul dazu konfiguriert ist, eine Position der physikalischen Bezugsmarkierung sowie eine Position des Bezugsbildes zu überwachen.

8. Lasersystem nach einem der vorangehenden Ansprüche, wobei zumindest entweder der gepulste Laser, das Bildgebermodul oder das Steuerungs-Modul dazu konfiguriert ist, eine Markierung auf einer intraokularen Linse oder einem kornealen Inlay in Verbidung mit der physikalischen Bezugsmarkierung zu verwenden, um entweder eine Positionierung, eine Auswahl einer optischen Stärke oder die Größe der intraokularen Linse oder des kornealen Inlays zu verbessern.

9. Lasersysteme nach einem der vorangehenden Ansprüche, aufweisend ein Computersystem zur Berechnung einer empfohlenen intraokularen Linsenstärke, die zumindest teilsweise auf der Position der physikalischen Bezugsmarkierung basiert.

## Revendications

1. Système laser pour chirurgie ophtalmique comprenant :
un laser pulsé (1010, 9100, 1710) adapté pour produire un faisceau d'impulsions laser (1012, 1022) ;
un mécanisme adapté pour aligner un oeil le long d'un axe sélectionné (330) de l'oeil ;
un module d'imagerie (1030, 2030, 2200, 2230, 3400, 5300) adapté pour surveiller au moins l'un du positionnement d'une marque de référence physique (350-1, 350-2) formée sur ou dans l'oeil, et une image de référence sélectionnée de la au moins une partie de l'oeil par rapport à l'axe sélectionné de l'oeil ; et
un module de commande (1040, 2020, 2120, 2220, 3100, 6100, 9200) en communication avec le module d'imagerie, adapté pour diriger les impulsions laser vers au moins une cible (1001, 1700) dans l'oeil par rapport à l'axe sélectionné de l'oeil,
le système laser étant configuré
- pour sélectionner un axe sur lequel aligner une procédure à impact optique ;
- pour former la marque de référence physique sur une région externe de l'oeil afin de représenter une position de l'axe sélectionné au moyen d'un laser ;
- pour identifier la marque de référence physique et déterminer la marque de référence physique identifiée sur une image de l'oeil ; et
- pour utiliser une position de la marque de référence physique identifiée sur l'image de l'oeil, afin de diriger des impulsions laser vers au moins une cible dans l'oeil par rapport à la marque de référence physique ou l'axe sélectionné de l'oeil,
**caractérisé en ce que** ledit système est en outre adapté pour placer la marque de référence physique sur ou dans l'oeil lorsque l'oeil est dans un état non aplani, et pour déterminer l'image de référence de la au moins une partie de l'oeil lorsque l'oeil est dans un état aplani.

2. Système laser selon la revendication 1, configuré en outre pour utiliser des marques de référence physiques (350-1, 350-2) générées par des impulsions laser afin de guider un alignement consécutif.

3. Système laser selon la revendication 1 ou 2, configuré en outre pour aligner des incisions (360) et des ablations cornéennes.

4. Système laser selon l'une des revendications précédentes, configuré en outre pour utiliser les marques de référence physique afin de guider l'alignement ou le placement d'un implant intraoculaire ou intracornéen.

5. Système laser selon l'une des revendications précédentes, configuré en outre pour utiliser des informations sur la marque de référence physique afin de faciliter la prédiction d'une position d'une lentille intraoculaire à implanter dans l'oeil.

6. Système laser selon l'une des revendications précédentes, dans lequel le mécanisme destiné à aligner l'oeil est configuré pour fixer une position de l'oeil par rapport à l'axe sélectionné.

7. Système laser selon l'une des revendications précédentes, dans lequel le module d'imagerie est configuré pour surveiller une position de la marque de référence physique et une position de l'image de référence.

8. Système laser selon l'une des revendications précédentes, dans lequel au moins l'un du laser pulsé, du module d'imagerie et du module de commande est configuré pour utiliser une marque sur une lentille intraoculaire ou un implant intracornéen conjointement avec la marque de référence physique, afin d'améliorer l'un d'un positionnement ou d'un choix de puissance optique ou de taille de la lentille intraoculaire ou de l'implant intracornéen.

9. Système laser selon l'une des revendications précédentes, comprenant un système informatique pour calculer une puissance de lentille intraoculaire recommandée sur la base, au moins en partie, de la position de la marque de référence physique.
